(19) Europäisches Patentamt
European Patent Office
Office européen des brevets

(11) Veröffentlichungsnummer: **0 150 677**
**B1**

(12) **EUROPÄISCHE PATENTSCHRIFT**

(45) Veröffentlichungstag der Patentschrift: **18.01.89**

(51) Int. Cl.⁴: **C 07 D 253/06**, C 07 C 133/10, A 01 N 43/64

(21) Anmeldenummer: **84810645.6**

(22) Anmeldetag: **19.12.84**

(54) **Herbizide und insektizide Triazinone.**

(30) Priorität: **11.01.84 CH 118/84**

(43) Veröffentlichungstag der Anmeldung: **07.08.85 Patentblatt 85/32**

(45) Bekanntmachung des Hinweises auf die Patenterteilung: **18.01.89 Patentblatt 89/3**

(84) Benannte Vertragsstaaten: **AT BE CH DE FR GB IT LI NL SE**

(56) Entgegenhaltungen:
**EP-A- 0 056 938**
**EP-A- 0 114 783**
**DD-A- 133 038**
**DE-A- 2 417 511**
**DE-B- 1 542 873**
**FR-A- 2 482 421**
**US-A- 3 905 801**

**CHEMICAL ABSTRACTS, Band 86, Nr. 13, 28. März 1977, Columbus, Ohio, USA; P. BARTL et al. "Ecological chemistry. CXIII. Photoinduced deamination reaction of 4-amino-3-methylthio-1,2,4-triazin-5(4H)-ones", Seite 455, linke Spalte, Zusammenfassung Nr. 88753g**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Böhner, Beat, Dr., Hügelweg 3, CH-4102 Binningen (CH)**
Erfinder: **Tobler, Hans, Dr., Baselmattweg 157, CH-4123 Allschwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft 4-Methylamino-1,2,4-triazin-5-one mit herbizider und insektizider Wirkung, Verfahren zu ihrer Herstellung, Mittel, die die neuen Wirkstoffe enthalten, und die Verwendung dieser 4-Methylamino-1,2,4-triazin-5-one oder sie enthaltender Mittel zur Bekämpfung von unerwünschtem Pflanzenwuchs und von schädlichen Insekten. Ferner betrifft die Erfindung zur Herstellung der 4-Methylamino-1,2,4-triazin-5-one entwickelte Zwischenprodukte sowie die Verfahren zu deren Herstellung.

Es sind bereits zahlreiche herbizid wirksame 4-Amino-1,2,4-Derivate bekannt, welche in 3-Position durch Mercapto, Alkylthio, Alkylamino oder Dialkylamino substituiert sind. Dagegen finden sich in der Literatur nur sehr vereinzelt Angaben über derartige Triazinone, bei denen die 4-Aminogruppe durch Methyl mono- oder disubstituiert ist. So werden beispielsweise in der US-A-3 671 523
3-Methylthio-4-dimethylamino-6-tert.butyl-1,2,4-triazin-5-on und
3-Mercapto-4-methylamino-6-phenyl-1,2,4-triazin-5-on
beschrieben, und aus der US-A-3 905 801 ist
3-Isobutylthio-4-methylamino-6-(2-naphthyl)-1,2,4-triazin-5-on
bekannt. Ferner beschreibt die japanische Patentpublikation 58/180 492 cyclische Analoge der vorgenannten Verbindungen, d.h., solche Verbindungen, bei denen die 3-Mercaptogruppe und die 4-Aminogruppe miteinander zur Ringstruktur verbunden sind, wie beispielsweise
3-(1,1-dimethylethyl)-4H-[1,3,4]thiadiazolo-[2,3-c]-[1,2,4]triazin-4-on.
Diese aus der Literatur bekannten und in 4-Position durch eine Methylamino- oder Dimethylaminogruppe substituierten 1,2,4-Triazinon-Derivate besitzen nur schwache herbizide Aktivität, welche sich als unzureichend für den Einsatz unter Praxisbedingungen erweist, zumal zum Teil bei gebräuchlichen Aufwandmengen keine herbizide Wirkung zu beobachten ist. Es muss daher als ausserordentlich überraschend angesehen werden, dass die neuen 3-Alkylamino- oder 3-Dialkylamino-4-methylamino-1,2,4-triazinon-Derivate, welche zwei einander unmittelbar benachbarte exocyclische Aminogruppen aufweisen, von denen diejenige in 4-Position durch eine Methylgruppe und diejenige in 3-Position durch Alkyl mono- oder disubstituiert ist, ausserordentlich starke Herbizide sind, die sich ausserdem teilweise durch selektive Eigenschaften in wichtigen Kulturen auszeichnen.

Die neuen 4-Methylamino-1,2,4-triazin-5-one sind zudem ausgezeichnet zur Bekämpfung schädlicher Insekten geeignet.

Insbesondere eignen sich die Verbindungen der Formel (I) und (III) zur Bekämpfung von Insekten der Ordnungen Lepidoptera, Coleoptera (insbesondere Blatt- und Rüsselkäfer), Homoptera (vor allem Zikaden und Blattläuse), Heteroptera, Diptera, Thysanoptera, Orthoptera, Anoplura, Siphonaptera, Mallophaga, Thysanura, Isoptera, Psocoptera und Hymenoptera.

Die gute insektizide Wirkung der erfindungsgemäss vorgeschlagenen Verbindungen der Vormel (I) und (III) entspricht einer Abtötungsrate (Mortalität) von mindestens 50–60% der erwähnten Schadinsekten.

Obwohl auf dem Gebiet der Triazinone eine intensive Forschung betrieben worden ist, welche zu zahlreichen Publikationen geführt hat, sind 3-Alkylamino- oder 3-Dialkylamino-4-methylamino-1,2,4-triazinon-Derivate mit Ausnahme des in der EP-A-114 783 beschriebenen
3,4-Bis-(methylamino)-6-(1,1-dimethyläthyl)-4H-1,2,4-triazin-5-on
bisher nicht bekannt geworden. Dies gilt auch für diejenigen erfindungsgemässen Verbindungen, bei denen die 3-Aminogruppe durch andere Kohlenwasserstoffreste substituiert ist.

Es wurde nun gefunden, dass 4-Methylamino-1,2,4-triazin-5-one der Erfindung ausgezeichnet zur Bekämpfung von unerwünschtem Pflanzenwuchs, insbesondere zur Bekämpfung von Unkräutern in Kulturpflanzenbeständen, geeignet sind.

Die erfindungsgemässen neuen 4-Methylamino-1,2,4-triazin-5-one entsprechen der Formel I

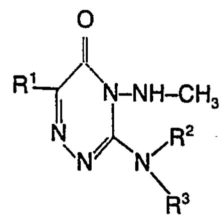
(I),

worin

$R^1$ für $C_3$–$C_8$-Cycloalkyl, durch $C_1$–$C_4$-Alkyl substituiertes $C_3$–$C_8$-Cycloalkyl, Halogen-$C_3$–$C_8$-cycloalkyl, $C_3$–$C_8$-Alkyl, Halogen-$C_3$–$C_8$-alkyl, 2-Furyl, 2-Thienyl, Phenyl, Benzyl oder durch Substituenten aus der Gruppe Halogen, $C_1$–$C_4$-Alkyl, Halogen-$C_1$–$C_4$-alkyl, $C_1$–$C_4$-Alkoxy oder Halogen-$C_1$–$C_4$-alkoxy substituiertes Phenyl oder Benzyl,

$R^2$ für Wasserstoff oder Methyl und

$R^3$ für Cyclopropyl, Cyclopropylmethyl, $C_1$–$C_4$-Alkyl, $C_3$–$C_4$-Alkenyl oder $C_3$–$C_4$-Alkinyl

stehen, wobei nicht gleichzeitig $R^1$ tert.Butyl, $R^2$ Wasserstoff und $R^3$ Methyl bedeuten dürfen.

Alkyl bedeutet in der angegebenen Definition je nach der Anzahl der Kohlenstoffatome beispielsweise Methyl, Äthyl, n-Propyl, i-Propyl, die vier isomeren Butyl sowie die isomeren Strukturen von Pentyl, Hexyl, Heptyl oder Oktyl. Alkyl steht dabei sowohl für den Substituenten Alkyl selbst als auch für einen Teil eines anderen Substituenten wie Alkoxy, Halogenalkyl oder Halogenalkoxy. Alkenyl und Alkinyl stehen im allgemeinen für Allyl, Methylallyl, 2-Butenyl, 3-Butenyl, Propargyl, 2-Butinyl oder 3-Butinyl. Bevorzugt sind Allyl und Propargyl. Cycloalkyl steht im Rahmen vorliegen-

der Erfindung für Cyclopropyl, Cyclobutyl, Cyclopentyl, Cyclohexyl, Cycloheptyl oder Cyclooctyl. Halogen als möglicher Substituent eines grösseren Restes steht für Fluor, Chlor, Brom oder Jod, wobei Fluor und Chlor bevorzugt sind. Durch Halogen substituierte Reste aus der Definition für $R^1$ können einfach bis vollständig halogeniert sein. So fallen beispielsweise unter den Begriff Halogencycloalkyl:
2-Chlorcyclopropyl, 3-Fluorcyclopentyl,
2-Chlorcyclopentyl, 2,3-Dichlorcyclopentyl,
2,3,4-Trichlorcyclopentyl, 3,4-Dichlorcyclopentyl,
3-Fluorcyclopentyl, 3-Bromcyclopentyl,
3-Fluorcyclohexyl, 4-Fluorcyclohexyl,
2-Chlorcyclohexyl, 3-Chlorcyclohexyl,
4-Chlorcyclohexyl, 2,6-Dichlorcyclohexyl,
2,4-Dichlorcyclohexyl,
2,3,4,5,6-Pentachlorcyclohexyl oder
3,4,5-Trichlorcyclohexyl;
unter den Begriff substituiertes Phenyl oder Benzyl:
2-Fluorphenyl, 2-Chlorphenyl,
2-Chlor-4-methoxyphenyl,
2-Chlor-4-trifluormethylphenyl,
2-Chlor-4-trifluormethoxyphenyl,
2,4-Dichlorphenyl, 2,6-Dichlorphenyl,
2,5-Dichlorphenyl, 3,4-Dichlorphenyl,
4-Chlorphenyl, 4-Trichlormethylphenyl,
4-Trifluormethylphenyl, 2-Trifluormethylphenyl,
4-Trifluormethoxyphenyl,
4-Difluormethoxyphenyl, 2-Methoxyphenyl,
2-Methylphenyl, 4-Methylphenyl,
4-Methoxyphenyl, 2,4-Di-methoxyphenyl,
2-Fluorbenzyl, 2-Chlorbenzyl,
2,6-Dichlorbenzyl oder 3,4-Dichlorbenzyl;
und unter den Begriff Halogenalkyl:
3,3,3-Trifluorpropyl, 1-Chlormethyläthyl,
1,1-Dimethyl-2-chloräthyl,
1,1-Dimethyl-2-fluoräthyl,
1,1-Dimethyl-3-chlorpropyl,
1-Methyl-3-chlorpropyl, 1-Methyl-3-fluorpropyl
oder 2,3-Dichlor-2-methylpropyl.

Wegen ihrer vorteilhaften biologischen Wirkung sind diejenigen Verbindungen der Formel (I) bevorzugt, in denen entweder
a) $R^1$ eine verzweigte $C_3$–$C_6$-Alkyl- oder eine verzweigte Halogen-$C_3$–$C_6$-alkylgruppe bedeutet oder
b) $R^2$ Wasserstoff bedeutet oder
c) $R^3$ für $C_1$–$C_4$-Alkyl steht,
mit der Massgabe, dass nicht gleichzeitig $R^1$ für tert.Butyl, $R^2$ für Wasserstoff und $R^3$ für Methyl stehen.

Innerhalb der Untergruppe a) sind die Verbindungen weiter bevorzugt, in denen $R^1$ eine verzweigte $C_3$–$C_6$-Alkylgruppe bedeutet und innerhalb der Untergruppe c) diejenigen, in denen $R^3$ für Methyl steht.

Weitere bevorzugte Untergruppen sind dadurch charakterisiert, dass $R^1$ für verzweigtes $C_3$–$C_6$-Alkyl oder verzweigtes Halogen-$C_3$–$C_6$-alkyl, $R^2$ für Wasserstoff oder Methyl und $R^3$ für $C_1$–$C_4$-Alkyl stehen, oder insbesondere dadurch, dass $R^1$ für verzweigtes $C_3$–$C_6$-Alkyl, $R^2$ für Wasserstoff und $R^3$ für Methyl stehen, wobei die vorstehend angegebene Massgabe gilt.

Ferner sind Verbindungen bevorzugt, in denen $R^1$ Phenyl oder durch Substituenten aus der Gruppe Halogen, $C_1$–$C_4$-Alkyl, Halogen-$C_1$–$C_4$-alkyl, $C_1$–$C_4$-Alkoxy oder Halogen-$C_1$–$C_4$-alkoxy substituiertes Phenyl bedeutet.

Als bevorzugte Einzelverbindungen der Formel (I) sind zu nennen:
3,4-Bis-(methylamino)-6-isopropyl-4H-1,2,4-triazin-5-on,
3-Dimethylamino-4-methylamino-6-(1,1-dimethyläthyl)-4H-1,2,4-triazin-5-on,
3,4-Bis-(methylamino)-6-(1-methylpropyl)-4H-1,2,4-triazin-5-on,
3,4-Bis-(methylamino)-6-phenyl-4H-1,2,4-triazin-5-on und
3,4-Bis-(methylamino)-6-(2-fluorphenyl)-4H-1,2,4-triazin-5-on.

Die Verbindungen der Formel (I) können nach den nachfolgend beschriebenen Verfahren hergestellt werden.

So erhält man die Verbindungen der Formel (I) beispielsweise, indem man entweder
a) ein 4-Amino-4H-1,2,4-triazin-5-on der Formel (II)

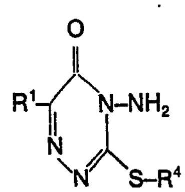

II

worin $R^1$ die unter Formel (I) gegebene Bedeutung hat und $R^4$ für Methyl oder Äthyl steht, mit einem Methylierungsmittel in ein 4-Methylamino-4H-1,2,4-triazin-5-on der Formel (III)

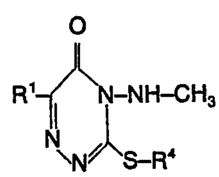

III

überführt und dieses bei erhöhter Temperatur mit einem Amin der Formel (IV)

$$H\text{–}NR^2R^3 \qquad IV$$

worin $R^2$ und $R^3$ die unter Formel (I) gegebenen Bedeutungen haben, umsetzt, mit der Massgabe, dass nicht gleichzeitig $R^2$ Wasserstoff und $R^3$ Methyl bedeuten, wenn $R^1$ für tert.Butyl steht; oder
b) ein 3,4-Diamino-4H-1,2,4-triazin-5-on der Formel (V)

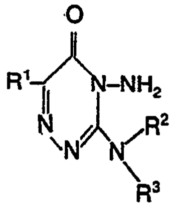

V

worin $R^1$, $R^2$ und $R^3$ die unter Formel (I) gegebenen Bedeutungen haben, mit einem Methylierungsmittel umsetzt, mit der Massgabe, dass nicht gleichzeitig $R^2$ Wasserstoff und $R^3$ Methyl bedeuten, wenn $R^1$ für tert.Butyl steht.

Bei diesem Verfahren werden die Methylierungsschritte (II) → (III) und (V) → (I) zweckmässigerweise unter Verwendung eines Methylhalogenids wie vorzugsweise Methylbromid oder Methyljodid oder von Dimethylsulfat in Gegenwart eines Phasentransferkatalysators in einem Zweiphasensystem durchgeführt. Es ist dabei vorteilhaft, die Umsetzung in Gegenwart eines organischen mit Wasser nicht mischbaren Lösungsmittels, einer wässrigen Lösung einer starken Base und eines Phasentransferkatalysators vorzunehmen, wobei es besonders günstig ist, in die Mischung aus dem organischen Lösungsmittel und der starken Base das Amin der Formeln (II) oder (V) einzutragen und dieses Gemisch mit Dimethylsulfat oder insbesondere einem Methylhalogenid und einem Phasentransferkatalysator, insbesondere einem quaternären Ammoniumsalz oder -hydroxid oder einem Phosphoniumsalz, zu versetzen. Als starke Basen kommen beispielsweise Alkalimetallhydroxide, wie Natrium- oder Kaliumhydroxid, oder Alkali- oder Erdalkalicarbonate in Betracht. Unter Methylhalogeniden sind z.B. Methylchlorid, Methylbromid und insbesondere Methyljodid zu verstehen. Als Ammoniumsalze oder -hydroxide kommen vorzugsweise solche aus der Gruppe Benzyltrialkylammonium- oder Tetraalkylammoniumhydroxid, -bisulfat oder -halogenid in Betracht, in denen die Alkylreste zweckmässigerweise 1 bis 4 Kohlenstoffatome enthalten, wie beispielsweise
Benzyltriäthylammoniumchlorid,
Tetra-n-butylammoniumhydroxid,
Benzyltrimethylammoniumchlorid.
Besonders geeignet ist ein Tetraalkylammoniumhalogenid, insbesondere Tetra-n-butylammoniumbromid. Beispiele für Phosphoniumsalze sind
Tributylhexadecylphosphonium-bromid,
Äthyltriphenylphosphonium-bromid,
Tetraphenylphosphonium-chlorid,
Benzyltriphenylphosphonium-jodid,
Triphenyl-n-propylphosphonium-bromid und
Tetrabutylphosphonium-chlorid.
Die Methylierungsreaktion lässt sich in einem breiteren Temperaturbereich durchführen, wobei jedoch ein Temperaturbereich zwischen 10° und 40 °C als besonders vorteilhaft anzusehen ist, und insbesondere ein Bereich von 20° bis 25 °C.

Die Umsetzung wird zweckmässigerweise in Gegenwart eines Lösungs- oder Verdünnungsmittels durchgeführt. Beispiele dafür sind aliphatische und aromatische Kohlenwasserstoffe, wie beispielsweise Benzol, Toluol, Xylole, Petroläther, Cyclohexan, n-Hexan; halogenierte Kohlenwasserstoffe wie Chlorbenzol, Methylenchlorid, Äthylenchlorid, Chloroform, Tetrachlorkohlenstoff, Tetrachloräthylen; Äther und ätherartige Verbindungen wie beispielsweise Dialkyläther, wie Diäthyläther, Diisopropyläther; oder Gemische solcher Lösungsmittel untereinander.

Der Aminierungsschritt (III) → (I) wird zweckmässigerweise bei erhöhter Temperatur, bevorzugt im Bereich von 100° bis 200 °C, insbesondere bei 140° bis 160 °C durchgeführt. Mit Vorteil führt man diese Reaktion in einem der bei der Methylierungsreaktion genannten inerten Lösungsmittel oder Lösungsmittelgemische aus.

Das bei dieser Umsetzung entstehende Methyl- oder Äthylmercaptan lässt sich aus dem Reaktionsgemisch leicht auf an sich bekannte Weise entfernen, beispielsweise durch Einleitung in Natriumhypochlorit.

Die Zwischenprodukte der Formel (III) sind – mit Ausnahme von 3-Methylthio-4-methylamino-6-tert.butyl-4,5-dihydro-1,2,4-triazin-5-on, welches aus Z. Naturforsch. 31b, 1122–1126 (1976), bekannt ist – neu und wurden speziell für die Synthese der Endprodukte der Formel (I) hergestellt. Sie bilden daher einen Bestandteil der vorliegenden Erfindung.

Die Zwischenprodukte der Formel (III) haben ebenfalls herbizide Eigenschaften. Die Verwendung dieser Verbindungen als Herbizide ist daher ebenfalls Gegenstand der Erfindung.

Die Ausgangsmaterialien der Formeln (II), (IV) und (V) sind bekannt und teilweise im Handel erhältlich.

Ferner können die Verbindungen der Formel (I) hergestellt werden, indem man ein α-Ketocarbonsäurederivat der Formel (VI)

$$R^1\text{–CO–A} \qquad \text{VI}$$

worin $R^1$ die unter Formel (I) gegebene Bedeutung hat und A für $-COOH$, $-COO-C_1-C_4$-Alkyl, $-COS-C_1-C_4$-Alkyl, $-CONH_2$, $-CONH-C_1-C_4$-Alkyl oder $CONH-CO-C_1-C_4$-Alkyl steht, mit einem Guanidinderivat der Formel (VII)

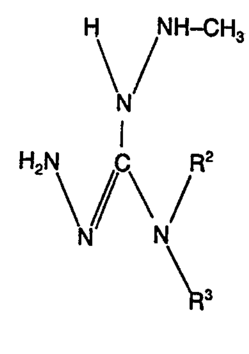

VII

worin $R^2$ und $R^3$ die unter Formel (I) gegebenen Bedeutungen haben, kondensiert.

Nach diesem Verfahren kann auch die unter Formel (I) ausgenommene Verbindung 3,4-Bis-(methylamino)-6-(1,1-dimethyläthyl)-4H-1,2,4-triazin-5-on hergestellt werden.

Die Kondensationsreaktion (VI) + (VII) → (I) wird mit Vorteil in Gegenwart einer katalytischen Menge einer organischen oder anorganischen Säure, wie Salzsäure, Schwefelsäure, Methansulfonsäure, Toluolsulfonsäure, Benzoesäure, Essigsäure, Trifluoressigsäure oder Bromwasserstoffsäure ausgeführt. Anstelle eines zusätzlichen Säurezusatzes kann auch direkt ein Säureadditionssalz des Guanidinderivates der Formel (VII), beispielsweise ein Hydrohalogenid wie das Hydrochlorid oder Hydrojodid, vorteilhaft eingesetzt werden. Die Reaktionstemperaturen liegen in der Regel zwischen 20 °C und 150 °C. Wird ein aktiviertes Carbonsäurederivat der Formel (VI), beispielsweise ein acyliertes Carbonsäureamid als Reaktionspartner eingesetzt, so kann die Reaktionstemperatur niedrig, z.B. bei 20 °C, gehalten werden. Höhere Temperaturen sind im allgemeinen für die Umsetzung von freien α-Ketocarbonsäuregruppen erforderlich. Vorzugsweise wird eine Temperatur im Bereich zwischen 50 °C und 90 °C gewählt. Ebenfalls von Vorteil ist bei der Kondensationsreaktion die Verwendung eines inerten Lösungsmittels wie Wasser, eines Alkohols wie Methanol, Äthanol, Äthylenglykol, Propanol, Isopropanol oder Butanol oder eines wasserlöslichen Äthers wie Tetrahydrofuran, Dioxan, Äthylenglykoldimethyläther oder Diäthylenglykoldimethyläther oder eines Gemisches solcher Lösungsmittel.

Die α-Ketocarbonsäurederivate der Formel (VI) sind bekannt und zum Teil im Handel erhältlich oder sie lassen sich analog zu bekannten Methoden herstellen.

Die Guanidinderivate der Formel (VII) sind neu und wurden speziell für die Synthese der Wirkstoffe der Formel (I) entwickelt und bereitgestellt. Sie bilden daher zusammen mit ihren Säureadditionssalzen einen Bestandteil der vorliegenden Erfindung.

Die Herstellung der neuen Guanidinderivate der Formel (VII) kann nach folgendem Schema 1 erfolgen:

Schema 1:

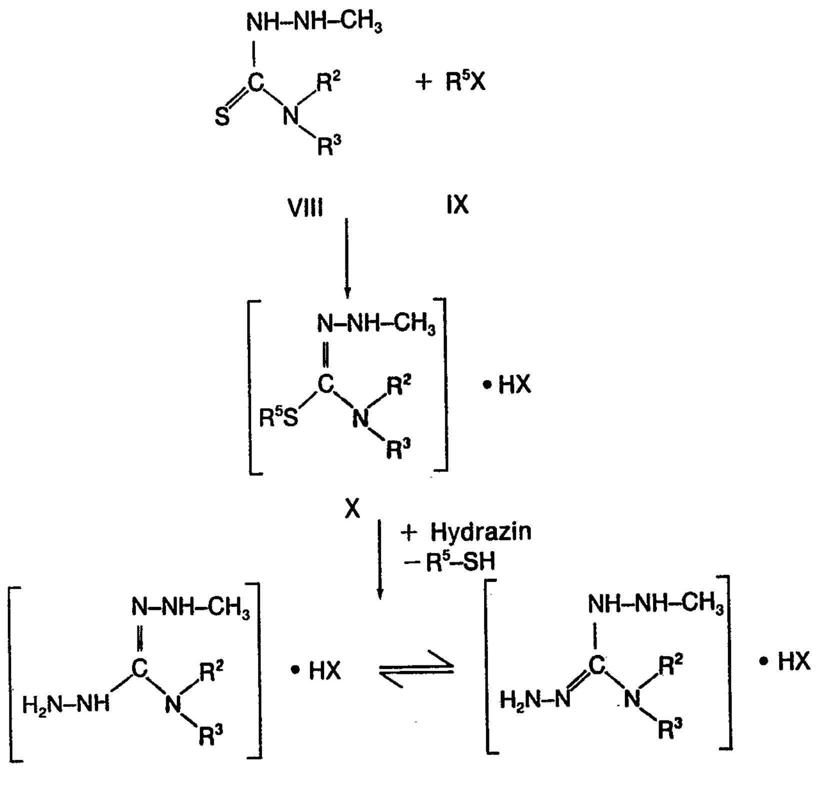


VIII IX

X

VIIa VIIb

Im Schema 1 haben $R^2$ und $R^3$ die unter Formel (I) gegebenen Bedeutungen, X steht für einen anorganischen oder starken organischen Säurerest und $R^5$ bedeutet $C_1$–$C_4$-Alkyl, vorzugsweise Methyl.

Nach dem obigen Schema 1 setzt man zur Herstellung der neuen Guanidinderivate der Formel (VII) zunächst ein Thiosemicarbazid der Formel (VIII) mit einem Alkylierungsmittel der Formel (IX) um und überführt das salzartige Zwischenprodukt der Formel (X) mit Hydrazin in das Guanidinderivat der Formel (VII), das zunächst in Form der tautomeren Säureadditionssalze der Formel (VIIa) und (VIIb) anfällt. Gewünschtenfalls können daraus die freien Verbindungen der Formel (VII) durch Behandlung mit einer Base erhalten werden. Für die Umsetzung der Guanidinderivate der Formel (VII) zu den Verbindungen der Formel (I) ist jedoch diese Freisetzung nicht notwendig, vielmehr ist es aus verfahrenstechnischen Gründen

von Vorteil, das Säureadditionssalz selbst in die Kondensationsreaktion (VII) + (VI) → (I) einzusetzen.

Starke Säuren, die die in den Alkylierungsmitteln $R^5X$ enthaltenen Molekülteile X liefern können, sind beispielsweise Chlorwasserstoff, Bromwasserstoff, Jodwasserstoff, Schwefelsäure, Methansulfonsäure oder Trifluormethansulfonsäure.

Die Ausgangsmaterialien der Formeln (VIII) und (IX) sind bekannt; die Alkylierungsmittel der Formel (IX) sind zum grossen Teil im Handel erhältlich.

Schliesslich kann man die Verbindungen der Unterformel (Ia)

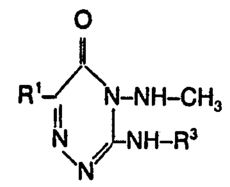

Ia

worin $R^1$ und $R^3$ die unter Formel (I) gegebenen Bedeutungen haben, auch durch gezielte Monomethylierung der 4-Aminogruppe aus den geeigneten 4-Amino-4H-1,2,4-triazin-5-onen der Formel (XI) nach dem folgenden Schema 2 herstellen:

Schema 2:

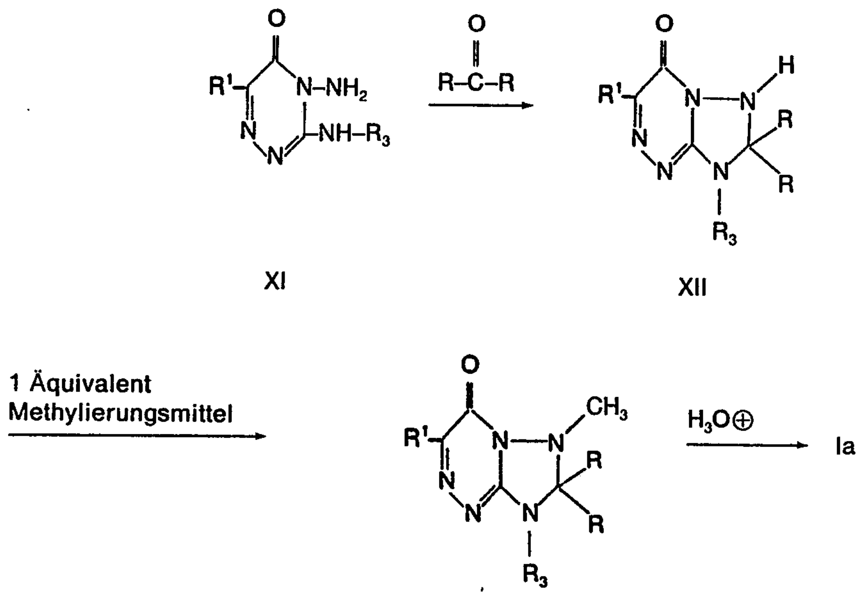


XI XII XIII

Im Schema 2 haben $R^2$ und $R^3$ die unter Formel (I) gegebenen Bedeutungen. R bedeutet $C_1$–$C_4$-Alkyl oder die Formel R–CO–R steht für ein carbocyclisches Keton wie Cyclopentanon oder Cyclohexanon. Das bevorzugt eingesetzte Keton ist jedoch Aceton oder 2-Butanon. Als Methylierungsmittel kommen vorzugsweise handelsübliche Verbindungen wie Dimethylsulfat, Methylchlorid, Methylbromid oder Methyljodid in Frage. Je nach Art des verwendeten Methylierungsreagenzes kann es zweckmässig sein, die Reaktion (XII) → (XIII) unter erhöhtem Druck (0 bis 20 bar) auszuführen.

Gemäss Schema 2 werden bei der angegebenen Reaktionsfolge zunächst die literaturbekannten 4-Amino-4H-1,2,4-triazin-5-one der Formel (XI) mit einem Keton in die bicyclische Struktur der Formel (XII) überführt und dann monomethyliert (Formel (XIII)). Durch eine saure Hydrolyse wird abschliessend das als Schutzgruppe eingeführte Keton wieder abgespalten, wodurch man den Wirkstoff der Formel (Ia) erhält, der durch zwei sekundäre Aminofunktionen gekennzeichnet ist.

Nach einer Variante dieses Verfahrens (XI) → (Ia) erhält man die Bis-methylamino-triazinone der Unterformel (Ib)

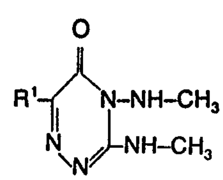

Ib

worin $R^1$ die unter Formel (I) gegebene Bedeutung hat, nach dem Schema 3 aus den entsprechenden 3,4-Diamino-4H-1,24-triazin-5-onen der Formel (XIV):

Schema 3:

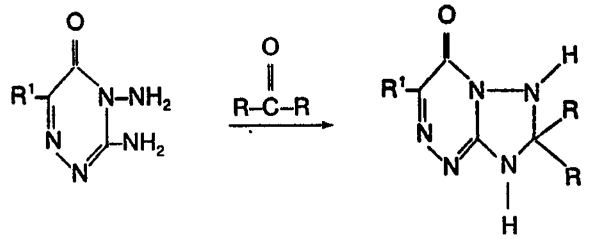

XIV XV

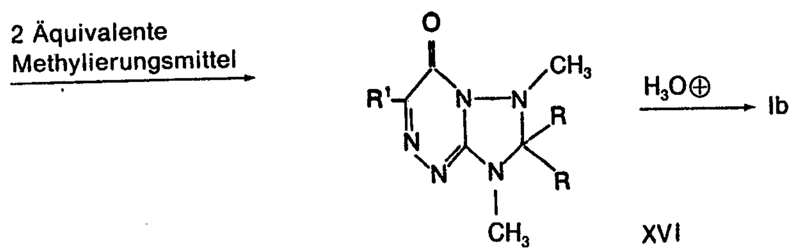


XVI

Die Reaktionsfolge im Schema 3 (XVI) → (Ib) wird analog zum Schema 2 geführt.

Die Ausgangsverbindungen (XI) und (XIV) sind aus der Literatur bekannt oder können analog zu bekannten Verfahren hergestellt werden.

Zur Applikation als Herbizid oder Insektizid werden die Wirkstoffe der Formeln (I) und (III) in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d. h., die die Wirkstoffe der Formeln (I) und (III) und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen des Wirkstoffs mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus können eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$–$C_{22}$), wie z. B. die Na- oder K-Salze der Öl- oder Stearinsäure, oder von natürlichen Fettsäuregemischen genannt, die z. B. aus Kokosnuss- oder Talgöl gewonnen werden können. Ferner sind auch die Fettsäuremethyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls

substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben:

«Mc Cutcheon's Detergents and Emulsifiers Annual» MC Publishing Corp., Ridgewood New Jersey, 1981;

H. Stache, «Tensid-Taschenbuch», 2. Aufl., C. Hanser Verlag, München, Wien, 1981;

M. and J. Ash. «Encyclopedia of Surfactants», Vol. I–III, Chemical Publishing Co., New York, 1980–1981.

Die agrochemischen Zubereitungen enthalten in der Regel 0,1 bis 99%, insbesondere 0,1 bis 95%, Wirkstoff, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Wirkstoffe der Formeln (I) und (III) zeigen eine ausgeprägte herbizide Aktivität. Sie sind zur Bekämpfung sowohl monokotyler wie dikotyler Unkräuter geeignet, und zwar sowohl bei der Anwendung im Vorauflaufverfahren (pre-emergent) wie im Nachauflaufverfahren (post-emergent).

Besonders vorteilhaft lassen sich die Wirkstoffe der Formeln (I) und (III) oder sie enthaltende Mittel zur selektiven Bekämpfung von Unkräutern in Nutzpflanzenkulturen einsetzen, wie beispielsweise in Kulturen von Getreide, Mais, Soja und insbesondere Zuckerrohr. Beim Einsatz in Zuckerrohr kann es zu einer beachtlichen Steigerung des Zuckergehalts kommen.

Die Aufwandmengen, in denen die neuen 4-Methylamino-1,2,4-triazin-5-one anzuwenden sind, hängen von den jeweiligen Gegebenheiten wie insbesondere vom Pflanzenbewuchs, der Bodenbeschaffenheit, den Witterungsbedingungen und dem Zeitpunkt der Applikation ab. Als zweckmässig erweisen sich im allgemeinen Aufwandmengen von 30 bis 2000 g/Hektar.

Herstellungsbeispiele:

Beispiel H1:

3,4-Bis-(methylamino)-6-phenyl-4H-1,2,4-triazin-5-on (Verb. Nr. 1.11)

a) 4-Methylamino-3-methylthio-6-phenyl-4H-1,2,4-triazin-5-on (Verb. Nr. 2.5).

Das zweiphasige Gemisch aus 35,1 g (0,15 Mol) 4-Amino-3-methylthio-6-phenyl-4H-1,2,4-triazin-5-on, 24,4 ml (0,375 Mol) Methyljodid, 5 g Tetrabutylammoniumbromid, 200 ml Toluol und 60 ml 50%iger Natronlauge wird für 2 Stunden kräftig gerührt. Dabei steigt die Temperatur von 20 °C auf 40 °C an. Die organische Phase wird abgetrennt und über Kieselgel filtriert. Durch Eindampfen des Filtrates erhält man 25,1 g (67,5% d. Th.) 4-Methylamino-3-methylthio-6-phenyl-4H-1,2,4-triazin-5-on, Smp. 119–120 °C.

b) 10,0 g (0,04 Mol) 4-Methylamino-3-methylthio-6-phenyl-4H-1,2,4-triazin-5-on, 1,6 g (0,05 Mol) Methylamin und 50 ml Isopropanol werden im Bombenrohr für 4 Stunden auf 155 °C erhitzt. Die erhaltene klare, orange Lösung wird eingedampft und mit einem Methylenchlorid/Äther-Gemisch (95:5)

an Kieselgel chromatographiert. Das Eluat wird eingedampft und der Rückstand mit Petroläther gewaschen. Man erhält so 8,1 g (88% d. Th.) 3,4-Bis-(methylamino)-6-phenyl-4H-1,2,4-triazin-5-on, Smp. 150–151 °C.

Beispiel H2:
3,4-Bis-(methylamino)-6-(3-fluorphenyl)-4H-1,2,4-triazin-5-on (Verb. Nr. 1.5).

a) 1,2-Dimethyl-3-methylamino-isothioharnstoff-hydrojodid.

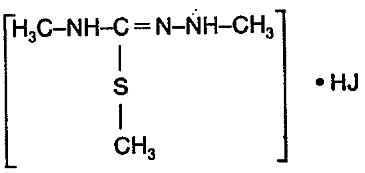

85,8 g (0,712 Mol) 1,4-Dimethylthiosemicarbazid ($H_3C–NH–CS–NH–NH–CH_3$), 103 g Methyljodid und 360 ml absolutes Äthanol werden für 7 Stunden im Autoklaven auf eine Temperatur zwischen 90 °C und 95 °C erhitzt. Der Druck beträgt dabei maximal 1 bar. Die nach dem Abkühlen erhaltene Suspension wird eingedampft und mit wenig kaltem Äthanol sowie Diäthyläther gewaschen. Man erhält so 161,7 g (86% d. Th.) 1,2-Dimethyl-3-methylamino-isothioharnstoff-hydrojodid als farbloses Kristallisat, Smp. 121–122 °C.

b) 1-Amino-2-methyl-3-methylamino-guanidin-hydrojodid.

161,7 g (0,6195 Mol) 1,2-Dimethyl-3-methylamino-isothioharnstoffhydrojodid werden in 850 ml Äthanol gelöst. Nachdem die Lösung auf 60 °C erwärmt worden ist, lässt man 21,5 g Hydrazin in 30 ml Äthanol langsam zutropfen. Aus der Lösung wird dabei Methylmercaptan freigesetzt. Zur Vervollständigung der Reaktion wird das Gemisch für 1,5 Stunden zum Rückfluss erhitzt. Nach der Beendigung der Gasentwicklung wird die Mischung abgekühlt und eingedampft. Der kristalline Rückstand wird mit Diethyläther gewaschen. Man erhält so 144,9 g (95,5% d. Th.)
1-Amino-2-methyl-3-methylamino-guanidin-hydrojodid, Smp. 175–177 °C.

c) Einer Lösung von 5,4 g (0,022 Mol) 1-Amino-2-methyl-3-methylamino-guanidin-hydrojodid in 55 ml 2N Salzsäure setzt man tropfenweise 3,8 g (0,02 Mol) 3-Fluorphenylglyoxylsäure in 10 ml Äthanol zu. Dabei steigt die Temperatur von 20 °C auf 30 °C an und es fällt ein blassgelber Niederschlag aus. Diese Mischung wird für zwei Stunden auf eine Temperatur zwischen 60 °C und 70 °C erhitzt, wobei eine klare gelborange Lösung entsteht. Nach dem Abkühlen wird die Lösung mit wässriger Natriumhydroxidlösung neutralisiert. Der ausfallende Niederschlag wird abgetrennt, mit Wasser gewaschen und bei einer Temperatur von 70 °C getrocknet. Man erhält so 4,3 g (76% d. Th.) 3,4-Bis-(methylamino)-6-(3-fluorphenyl)-4H-1,2,4-triazin-5-on, Smp. 173–174 °C.

Beispiel H3:
3,4-Bis-(methylamino)-6-(1,1-dimethyläthyl)-4H-1,2,4-triazin-5-on

a) 3-(1,1-Dimethyläthyl)-7,7,8-trimethyl-5,6,7,8-tetrahydro-5H[1,2,4]triazolo-[5,1-c][1,2,4]triazin-4-on

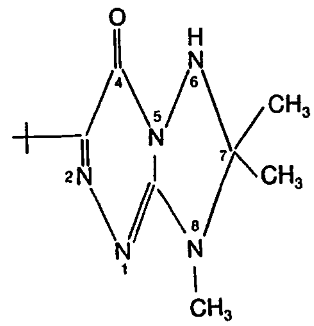

19,7 g (0,082 Mol) 4-Amino-3-methylamino-6-(1,1-dimethyläthyl)-4H-1,2,4-triazin-5-on werden zusammen mit 50 ml Aceton und 50 ml Eisessig für eine Stunde zum Rückfluss erhitzt. Anschliessend wird das Reaktionsgemisch eingedampft und der Rückstand aus einem Äther/Petroläther-Gemisch kristallisiert. Man erhält so 21,6 g (91% d. Th.)
3-(1,1-Dimethyläthyl)-7,7,8-trimethyl-5,6,7,8-tetrahydro-5H[1,2,4]triazolo[5,1-c][1,2,4]-triazin-4-on, Smp. 165–168 °C.

b) 3-(1,1-Dimethyläthyl)-6,7,7,8-tetramethyl-5,6,7,8-tetrahydro-5H[1,2,4]triazolo[5,1-c]-[1,2,4]triazin-4-on.

Das zweiphasige Gemisch aus 11,0 g (0,046 Mol)
3-(1,1-Dimethyläthyl)-7,7,8-trimethyl-5,6,7,8-tetrahydro-5H-[1,2,4]triazolo[5,1-c][1,2,4]-triazin-4-on,
3,0 g Tetrabutylammoniumbromid, 12,5 ml (0,2 Mol) Methyljodid, 25 ml Toluol und 20 ml 50%iger Natronlauge wird für eine Stunde kräftig gerührt. Dabei steigt die Temperatur von 20 °C auf 40 °C an. Zur Vervollständigung der Reaktion erwärmt man das Gemisch auf 50 °C und rührt für eine weitere Stunde. Die organische Phase wird abgetrennt und eingedampft. Durch Kristallisation des Rückstandes aus Äthylacetat erhält man 7,4 g (63,8% d. Th.)
3-(1,1-Dimethyläthyl)-6,7,7,8-tetramethyl-5,6,7,8-tetrahydro-5H-[1,2,4]triazolo-[5,1-c][1,2,4]triazin-4-on, Smp. 120–121 °C.

c) 2,5 g (0,01 Mol)
3-(1,1-Dimethyläthyl)-6,7,7,8-tetramethyl-5,6,7,8-tetrahydro-5H-[1,2,4]triazolo[5,1-c]-[1,2,4]triazin-4-on
werden in 10 ml Isopropanol gelöst und mit 0,15 g p-Toluolsulfonsäure versetzt. Bei einer Temperatur von 60 °C werden 0,72 ml Wasser und 10 ml Äthylenglykolmonomethyläther zugegeben. Dann wird das Reaktionsgemisch auf einem Ölbad bis zu einer Temperatur von 155 °C erhitzt. Nach 10 Minuten ist im Dünnschichtchromatogramm kein Ausgangsmaterial mehr nachweisbar. Das Reaktionsgemisch wird bis zur Trockne eingedampft. Der ölige Rückstand wird in Methylenchlorid gelöst und über Kieselgel filtriert. Durch Eindampfen des Filtrats und Kristallisation aus Äther/Petroläther erhält man 2,0 g (95,2% d. Th.)
3,4-Bis-(methylamino)-6-(1,1-Dimethyläthyl)-4H-1,2,4-triazin-5-on, Smp. 170–171 °C.

Beispiel H4:
3,4-Bis-(methylamino)-6-(1,1-dimethyläthyl)-4H-1,2,4-triazin-5-on

a) 3,4-Diamino-6-(1,1-dimethyläthyl)-4H-1,2,4-triazin-5-on.

3,8 g (0,03 Mol) Diaminoguanidinhydrochlorid werden in 35 ml Wasser gelöst. Dieser Lösung setzt man 5,1 g (0,03 Mol) N-Acetyl-3,3,3-trimethylbrenztraubensäureamid zu und heizt das Reaktionsgemisch für 15 Minuten auf 90 °C auf. Das Reaktionsgemisch wird filtriert und durch Zusatz von kristallinem Natriumhydrogencarbonat neutralisiert. Bei pH 7 fallen 3,6 g (66,7% d. Th.)
3,4-Diamino-6-(1,1-dimethyläthyl)-4H-1,2,4-triazin-5-on als gelbstichiges Kristallisat an, Smp. 223–224 °C.

b) 3-(1,1-Dimethyläthyl)-7,7-dimethyl-5,6,7,8-tetrahydro-5H-[1,2,4]triazolo[5,1-c]-[1,2,4]triazin-4-on.

7,0 g (0,0382 Mol) 3,4-Diamino-6-(1,1-dimethyläthyl)-4H-1,2,4-triazin-5-on werden in 150 ml Aceton am Rückfluss gekocht. Nach einer Stunde entsteht eine klare Lösung. Diese Lösung wird mit 4 g festem Natriumhydroxid versetzt und bei einer Temperatur von 50 °C für ca. 30 Minuten gerührt. Danach ist dünnschichtchromatographisch kein Ausgangsmaterial mehr nachweisbar. Nach dem Abkühlen filtriert man 10,5 g eines gelben Niederschlages ab. Bei der Filtration achtet man darauf, dass das feste Natriumhydroxid im Reaktionsgefäss zurückbleibt. Das abgetrennte gelbe Produkt wird in 50 ml Wasser gelöst und mit 36%iger Salzsäure neutralisiert. Dabei entfärbt sich die klare gelbe Lösung. Bei pH 7 fällt ein farbloser Niederschlag aus. Durch Isolierung dieses Kristallisats erhält man 5,0 g (59,5% d. Th.)
3-(1,1-Dimethyläthyl)-7,7-dimethyl-5,6,7,8-tetrahydro-5H-[1,2,4]triazolo[5,1-c][1,2,4]-triazin-4-on, Smp. 170–172 °C.

c) 3-(1,1-Dimethyläthyl)-6,7,7,8-tetramethyl-5,6,7,8-tetrahydro-5H-[1,2,4]triazolo[5,1-c]-[1,2,4]triazin-4-on.

Das zweiphasige Gemisch aus 4,9 g (0,022 Mol)
3-(1,1-Dimethyläthyl)-7,7-dimethyl-5,6,7,8-tetrahydro-5H-[1,2,4]triazolo[5,1-c][1,2,4]-triazin-4-on,
1,5 g Tetrabutylammoniumbromid, 6,2 ml (0,1 Mol) Methyljodid, 12 ml Toluol und 10 ml 50%iger Natronlauge wird für eine Stunde kräftig gerührt. Das Reaktionsgemisch erwärmt sich dabei von 20 °C auf 30 °C. Anschliessend erhitzt man die Mischung für weitere 15 Minuten auf 40 °C und extrahiert das Reaktionsgemisch mit Äthylacetat. Durch Eindampfen der organischen Phase erhält man 5,9 g eines orangeroten Öls, das noch 12,7% eines Isomeren enthält. Dieses Isomere wird durch Säulenchromatographie entfernt. Die Hauptfraktion wird eingedampft und aus Äther/Petroläther kristallisiert. Man erhält so das reine
3-(1,1-Dimethyläthyl)-6,7,7,8-tetramethyl-5,6,7,8-tetrahydro-5H-[1,2,4]triazolo[5,1-c]-[1,2,4]triazin-4-on, Smp. 120–121 °C.

d) 1,25 g (0,005 Mol)
3-(1,1-Dimethyläthyl)-6,7,7,8-tetramethyl-5,6,7,8-tetrahydro-5H-[1,2,4]triazolo[5,1-c]-[1,2,4]triazin-4-on
werden in 10 ml 1N Salzsäure suspendiert und unter Rühren auf 90 °C erhitzt. Nach 15 Minuten ist in der Mischung im Dünnschichtchromatogramm kein Ausgangsmaterial mehr nachweisbar. Nach dem Abkühlen wird die Mischung mit 1N Natronlauge neutralisiert. Dabei fallen 0,5 g (50% d. Th.)
3,4-Bis-(methylamino)-6-(1,1-dimethyläthyl)-4H-1,2,4-triazin-5-on an, Smp. 170–172 °C.

In analoger Weise erhält man die in den anschliessenden Tabellen zusammen mit den Verbindungen der Formeln (I) und (III) der vorstehenden Beispiele ausgeführten Zwischen- und Endprodukte.

Tabelle 1

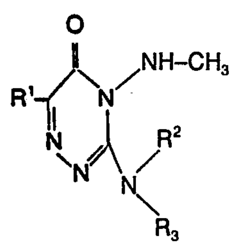

| Verb. Nr. | $R^1$ | $R^2$ | $R^3$ | phys. Daten |
|---|---|---|---|---|
| 1.1 | $C_4H_9$–t | $CH_3$ | $CH_3$ | Smp. 69–70 °C |
| 1.2 | $C_4H_9$–s | H | $CH_3$ | Smp. 93–94 °C |
| 1.3 | $C_4H_9$–t | H | $C_3H_7$–n | Smp. 43–47 °C |
| 1.4 | $C_4H_9$–t | H | $C_3H_7$–i | Smp. 72–74 °C |
| 1.5 | 3–F–$C_6H_4$– | H | $CH_3$ | Smp. 173–174 °C |
| 1.6 | $C_4H_9$–t | H | $CH_2CH = CH_2$ | Öl |

Tabelle 1 (Fortsetzung)

| Verb. Nr. | $R^1$ | $R^2$ | $R^3$ | phys. Daten |
|---|---|---|---|---|
| 1.7 | $C_4H_9$–t | H | $C_3H_5$–cycl. | Smp. 165–166 °C |
| 1.8 | $C_3H_7$–i | H | $CH_3$ | Smp. 117–118 °C |
| 1.9 | $C_6H_{11}$–cycl. | H | $CH_3$ | Smp. 121–122 °C |
| 1.10 | $C_4H_9$–t | H | $C_4H_9$–n | Smp. 79–80 °C |
| 1.11 | $C_6H_5$– | H | $CH_3$ | Smp. 150–151 °C |
| 1.12 | 3–Cl–$C_6H_4$– | H | $CH_3$ | Smp. 136–138 °C |
| 1.13 | 3–$CF_3$–$C_6H_4$ | H | $CH_3$ | viskos |
| 1.14 | 3–$CH_3$–$C_6H_4$– | H | $CH_3$ | Smp. 123–125 °C |
| 1.15 | 4–Cl–$C_6H_4$– | H | $CH_3$ | Smp. 168–169 °C |
| 1.16 | 4–$CF_3$–$C_6H_4$ | H | $CH_3$ | Smp. 191–192 °C |
| 1.17 | $ClCH_2$–$C(CH_3)_2$– | H | $CH_3$ | Smp. 110–111 °C |
| 1.18 | $ClCH_2$–$C(CH_3)_2$– | $CH_3$ | $CH_3$ | |
| 1.19 | 3–$CF_3$–$C_6H_4$– | $CH_3$ | $CH_3$ | Smp. 132–133 °C |
| 1.20 | 3–CL–$C_6H_4$– | $CH_3$ | $CH_3$ | Smp. 99–101 °C |
| 1.21 | 3–F–$C_6H_4$– | $CH_3$ | $CH_3$ | Smp. 89–90 °C |
| 1.22 | $C_6H_5$– | $CH_3$ | $CH_3$ | Smp. 88–89 °C |
| 1.23 | $C_6H_{11}$–cycl. | $CH_3$ | $CH_3$ | |
| 1.24 | $C_3H_7$–i | $CH_3$ | $CH_3$ | $n_D^{22}$ = 1.5366 |
| 1.25 | $C_4H_9$–s | $CH_3$ | $CH_3$ | $n_D^{22}$ = 1.5297 |
| 1.26 | $C_3H_7$–$C(CH_3)_2$– | H | $CH_3$ | |
| 1.27 | $C_3H_7$–$C(CH_3)_2$– | $CH_3$ | $CH_3$ | |
| 1.28 | $(CH_3)_3C$–$CH_2$– | H | $CH_3$ | |
| 1.29 | $(CH_3)_3C$–$CH_2$– | $CH_3$ | $CH_3$ | Smp. 91–92 °C |
| 1.30 | 1-Methylcyclopentyl | H | $CH_3$ | |
| 1.31 | 1-Methylcyclopentyl | $CH_3$ | $CH_3$ | |
| 1.32 | 1-Methylcyclohexyl | H | $CH_3$ | |
| 1.33 | 1-Methylcyclohexyl | $CH_3$ | $CH_3$ | |
| 1.34 | 1-Äthylcyclopentyl | H | $CH_3$ | |
| 1.35 | 1-Äthylcyclopentyl | $CH_3$ | $CH_3$ | |
| 1.36 | 3–Br–$C_6H_4$– | H | $CH_3$ | Smp. 138–141 °C |
| 1.37 | $C_4H_9$–t | H | $C_2H_5$ | Smp. 126–127 °C |
| 1.38 | $C_6H_5$ | H | $C_2H_5$ | Smp. 133–134 °C |
| 1.39 | 2–Thienyl | H | $CH_3$ | Smp. 190–191 °C |
| 1.40 | $(CH_3)_2CH$–$CH_2$ | H | $CH_3$ | Smp. 119–120 °C |
| 1.41 | 3–Br–$C_6H_4$ | $CH_3$ | $CH_3$ | Smp. 133–134 °C |
| 1.42 | 3–$CH_3$–$C_6H_4$ | $CH_3$ | $CH_3$ | Smp. 88–90 °C |
| 1.43 | 4–Cl–$C_6H_4$ | $CH_3$ | $CH_3$ | Smp. 118–120 °C |
| 1.44 | 2–F–$C_6H_4$ | H | $CH_3$ | Smp. 145–146 °C |
| 1.45 | 4–$CH_3$–$C_6H_4$ | $CH_3$ | $CH_3$ | Smp. 102–103 °C |
| 1.46 | 2–F–$C_6H_4$ | $CH_3$ | $CH_3$ | Smp. 79–81 °C |
| 1.47 | 2–$CH_3$–$C_6H_4$ | H | $CH_3$ | Smp. 120–122 °C |
| 1.48 | $(CH_3)_2CH$–$CH_2$– | $CH_3$ | $CH_3$ | $n_D^{22}$: 1.5316 |
| 1.49 | $(C_2H_5)_2CH$– | $CH_3$ | $CH_3$ | $n_D^{22}$: 1.5164 |
| 1.50 | 2–$CH_3$–$C_6H_4$ | $CH_3$ | $CH_3$ | Smp. 122–124 °C |
| 1.51 | 4–$CH_3$–$C_6H_4$ | H | $CH_3$ | Smp. 160–162 °C |
| 1.52 | $C_3H_7$–$CH(CH_3)$– | $CH_3$ | $CH_3$ | $n_D^{22}$: 1.5273 |
| 1.53 | 3–$CH_3$–$C_6H_4$–$CH_2$ | H | $CH_3$ | Smp. 96–98 °C |
| 1.54 | 2–F, 6–F–$C_6H_3$ | H | $CH_3$ | Smp. 193–195 °C |
| 1.55 | 2–F, 6–F–$C_6H_3$ | $CH_3$ | $CH_3$ | |
| 1.56 | 2–Cl, 6–Cl–$C_6H_3$ | H | $CH_3$ | |
| 1.57 | 2–Cl, 6–Cl–$C_6H_3$ | $CH_3$ | $CH_3$ | |
| 1.58 | 2–$C_2H_5$–$C_6H_4$ | H | $CH_3$ | Smp. 70–74 °C |
| 1.59 | 2–$C_2H_5$–$C_6H_4$ | $CH_3$ | $CH_3$ | |
| 1.60 | 2–$CH_3$, 6–$CH_3$–$C_6H_3$ | H | $CH_3$ | |
| 1.61 | 2–$CH_3$, 6–$CH_3$–$C_6H_3$ | $CH_3$ | $CH_3$ | |
| 1.62 | 2–$C_3H_7$–i–$C_6H_4$ | H | $CH_3$ | |
| 1.63 | 2–$C_3H_7$–i–$C_6H_4$ | $CH_3$ | $CH_3$ | |
| 1.64 | 2–Cl–$C_6H_4$ | H | $CH_3$ | |
| 1.65 | 2–Cl–$C_6H_4$ | $CH_3$ | $CH_3$ | |
| 1.66 | 3–Cl, 4–Cl–$C_6H_3$ | H | $CH_3$ | Smp. 207–209 °C |
| 1.67 | 2–Cl, 4–Cl–$C_6H_3$ | H | $CH_3$ | Smp. 178–180 °C |

Tabelle 2

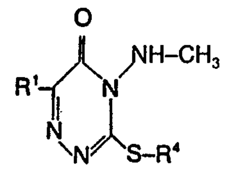

| Verb. Nr. | $R^1$ | $R^4$ | phys. Daten |
|---|---|---|---|
| 2.1 | $C_4H_9$–s | $CH_3$ | Öl, $n_D^{22}$ = 1.5598 |
| 2.2 | $C_6H_{11}$–cycl. | $CH_3$ | Öl |
| 2.3 | $C_3H_7$–i | $CH_3$ | Smp. 79–80 °C |
| 2.4 | $C_4H_9$–i | $CH_3$ | Smp. 71–73 °C |
| 2.5 | $C_6H_5$ | $CH_3$ | Smp. 119–120 °C |
| 2.6 | 3–F–$C_6H_4$ | $CH_3$ | Smp. 173–175 °C |
| 2.7 | 4–Cl–$C_6H_4$ | $CH_3$ | Smp. 204–206 °C |
| 2.8 | 3–Cl–$C_6H_4$ | $CH_3$ | Smp. 195–197 °C |
| 2.9 | 3–Br–$C_6H_4$ | $CH_3$ | Smp. 204–205 °C |
| 2.10 | 3–$CH_3$–$C_6H_4$ | $CH_3$ | Smp. 154–156 °C |
| 2.11 | 3–$CF_3$–$C_6H_4$ | $CH_3$ | Smp. 170–172 °C |
| 2.12 | $C_6H_5$ | $C_2H_5$ | Smp. 125–126 °C |
| 2.13 | $C_6H_{11}$ cycl. | $C_2H_5$ | $n_D^{23}$: 1.5574 |
| 2.14 | $C_4H_9$–t | $C_2H_5$ | Smp. 61–62 °C |
| 2.15 | $C_3H_7$–i | $C_2H_5$ | Smp. 71–72 °C |
| 2.16 | $C_4H_9$–s | $C_2H_5$ | $n_D^{23}$: 1.5466 |
| 2.17 | $(C_2H_5)_2CH$– | $C_2H_5$ | $n_D^{23}$: 1.5457 |
| 2.18 | 2–F–$C_6H_4$ | $CH_3$ | Smp. 96–98 °C |
| 2.19 | 4–$CH_3$–$C_6H_4$ | $CH_3$ | Smp. 165–166 °C |
| 2.20 | $(C_2H_5)_2CH$– | $CH_3$ | $n_D^{22}$: 1.5448 |
| 2.21 | 2–$CH_3$–$C_6H_4$ | $CH_3$ | Smp. 131–133,5 °C |
| 2.22 | $CH_3$–$CH_2CH_2$–CH– <br> \| <br> ($CH_3$) | $CH_3$ | $n_D^{22}$: 1.5520 |
| 2.23 | 2–Furyl | $CH_3$ | Smp. 170–171 °C |
| 2.24 | $(CH_3)_3C$–$CH_2$– | $CH_3$ | Smp. 88–89 °C |
| 2.25 | 2–F, 6–F–$C_6H_3$ | $CH_3$ | Smp. 156–158 °C |
| 2.26 | 2–Cl, 6–Cl–$C_6H_3$ | $CH_3$ | |
| 2.27 | 2–$CH_3$, 6–$CH_3$–$C_6H_3$ | $CH_3$ | |
| 2.28 | 2–$C_2H_5$–$C_6H_4$ | $CH_3$ | |
| 2.29 | 2–$C_3H_7$–i–$C_6H_4$ | $CH_3$ | |
| 2.30 | 2–Cl–$C_6H_4$ | $CH_3$ | |

Formulierungsbeispiele:
(% = Gewichtsprozent)

| Beispiel F1: Spritzpulver | a) | b) | c) |
|---|---|---|---|
| Wirkstoff der Formel (I) oder (III) | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyäthylenglykoläther (7–8 Mol AeO) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Beispiel F2:
Emulsions-Konzentrat

| | |
|---|---|
| Wirkstoffe der Formel (I) oder (III) | 10% |
| Octylphenolpolyäthylenglykoläther (4–5 Mol AeO) | 3% |
| Ca-Dodecylbenzolsulfonat | 3% |
| Ricinusölpolyglykoläther (35 Mol AeO) | 4% |
| Dimethylformamid | 20% |
| Cyclohexanon | 20% |
| Xylolgemisch | 40% |

Aus diesem Konzentrat können durch Verdünnen mit Wasser Emulsionen jeder gewünschten Konzentration hergestellt werden.

| Beispiel F3: Stäubemittel | a) | b) |
|---|---|---|
| Wirkstoff der Formel I oder III | 5% | 8% |
| Talkum | 95% | – |
| Kaolin | – | 92% |

Man erhält anwendungsfertige Stäubemittel, indem der Wirkstoff mit den Trägerstoffen vermischt und auf einer geeigneten Mühle vermahlen wird.

Beispiel F4:
Extruder-Granulat

| | |
|---|---|
| Wirkstoffe der Formel (I) oder (III) | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

Beispiel F5:
Umhüllungs-Granulat

| | |
|---|---|
| Wirkstoff der Formel (I) oder (III) | 3% |
| Polyäthylenglykol (MG 200) | 3% |
| Kaolin, gekörnt | 94% |

Der fein gemahlene Wirkstoff wird in einem Mischer auf das mit Polyäthylenglykol angefeuchtete, gekörnte Kaolin gleichmässig aufgetragen. Auf diese Weise erhält man staubfreie Umhüllungs-Granulate.

Beispiel F6:
Suspensions-Konzentrat

| | |
|---|---|
| Wirkstoff der Formel (I) oder (III) | 40% |
| Äthylenglykol | 10% |
| Nonylphenolpolyäthylenglykoläther (15 Mol AeO) | 6% |
| Na-Ligninsulfonat | 10% |
| Carboxymethylcellulose | 1% |
| 37%-ige wässrige Formaldehyd-Lösung | 0,2% |
| Silikonöl in Form einer 75%-igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel B1:
Pre-emergente Herbizid-Wirkung

Im Gewächshaus wird unmittelbar nach der Einsaat der Versuchspflanzen in Töpfe von 11 cm Durchmesser die Erdoberfläche mit einer wässrigen Dispersion eines Wirkstoffs der Formel (I) oder (III), erhalten aus einem 25%igen Spritzpulver oder einem 25%igen Emulsionskonzentrat, behandelt. Es werden Konzentrationen von 4 kg/Hektar angewendet. Die angesäten Töpfe werden im Gewächshaus bei 20 bis 24 °C und 50 bis 70% relativer Luftfeuchtigkeit gehalten. Der Versuch wird nach 3 Wochen ausgewertet und die Resultate werden nach folgender Notenskala bonitiert:

1 = Pflanzen nicht gekeimt oder total abgestorben
2–3 = sehr starke Wirkung
4–6 = mittlere Wirkung
7–8 = geringe Wirkung
9 = keine Wirkung (wie unbehandelte Kontrolle)

Die Resultate sind wie folgt:

Tabelle 3
Pre-emergente Wirkung
Aufwandmenge: 4 kg Wirksubstanz/Hektar

| Verb. Nr. | Avena | Setaria | Sinapis | Stellaria |
|---|---|---|---|---|
| 1.1 | 1 | 1 | 1 | 1 |
| 1.2 | 1 | 1 | 1 | 2 |
| 1.3 | 2 | 1 | 1 | 2 |
| 1.4 | 1 | 1 | 1 | 2 |
| 1.5 | 2 | 1 | 1 | 2 |
| 1.6 | 1 | 1 | 1 | 1 |
| 1.7 | 2 | 1 | 1 | 1 |
| 1.8 | 1 | 1 | 1 | 2 |
| 1.9 | 2 | 1 | 1 | 1 |
| 1.11 | 1 | 1 | 1 | 2 |
| 1.12 | 1 | 1 | 1 | 2 |
| 1.13 | 1 | 1 | 1 | 1 |
| 1.14 | 1 | 1 | 1 | 2 |
| 1.15 | 2 | 1 | 1 | 1 |
| 1.16 | 2 | 1 | 1 | 1 |
| 1.17 | 2 | 1 | 1 | 1 |
| 1.19 | 1 | 1 | 1 | 1 |
| 1.20 | 2 | 1 | 1 | 2 |
| 1.21 | 1 | 1 | 1 | 1 |
| 1.22 | 1 | 1 | 1 | 1 |
| 1.24 | 1 | 1 | 1 | 1 |
| 1.25 | 1 | 1 | 1 | 1 |
| 1.36 | 2 | 1 | 1 | 1 |
| 1.37 | 2 | 1 | 1 | 2 |
| 1.38 | 1 | 1 | 1 | 1 |
| 1.39 | 1 | 1 | 1 | 1 |
| 1.40 | 1 | 1 | 1 | 1 |
| 1.41 | 1 | 2 | 1 | 1 |
| 1.42 | 1 | 1 | 1 | 1 |
| 1.43 | 2 | 1 | 1 | 2 |
| 1.44 | 1 | 1 | 1 | 1 |
| 1.45 | 1 | 1 | 1 | 4 |
| 1.46 | 1 | 1 | 1 | 1 |
| 1.47 | 1 | 1 | 1 | 1 |
| 1.48 | 1 | 1 | 1 | 1 |
| 1.49 | 1 | 1 | 1 | 1 |
| 1.50 | 1 | 1 | 1 | 1 |
| 1.51 | 1 | 1 | 1 | 1 |
| 2.1 | 1 | 1 | 1 | 2 |
| 2.2 | 2 | 1 | 1 | 1 |
| 2.3 | 1 | 1 | 1 | 2 |
| 2.4 | 2 | 1 | 1 | 2 |
| 2.5 | 2 | 1 | 1 | 1 |
| 2.6 | 1 | 1 | 1 | 1 |
| 2.10 | 1 | 1 | 1 | 1 |
| 2.11 | 1 | 7 | 2 | 2 |

Tabelle 3 (Fortsetzung)

| Verb. Nr. | Avena | Setaria | Sinapis | Stellaria |
|---|---|---|---|---|
| 2.12 | 8 | 6 | 9 | 6 |
| 2.13 | 1 | 1 | 1 | 1 |
| 2.14 | 8 | 1 | 2 | 5 |
| 2.16 | 2 | 1 | 1 | 1 |
| 2.17 | 2 | 3 | 2 | 1 |
| 2.18 | 1 | 1 | 1 | 1 |
| 2.19 | 5 | 4 | 1 | 1 |
| 2.20 | 1 | 1 | 1 | 1 |
| 2.21 | 1 | 1 | 1 | 1 |
| 2.22 | 1 | 1 | 1 | 1 |
| 2.23 | 7 | 1 | 9 | 5 |

Beispiel B2:

Post-emergente Herbizid-Wirkung

Im Gewächshaus werden in Töpfen von 11 cm Durchmesser die Pflanzen Soja, Avena fatua, Setaria, Lolium, Solanum, Stellaria, Sinapis und Phaseolus gezogen, bis sie das 3–6-Blatt-Stadium erreicht haben, was nach ca. 2 Wochen der Fall ist. Dann werden sie mit einer wässrigen Wirkstoffemulsion oder einem Spritzpulver in einer Dosierung von 4 kg Wirksubstanz pro Hektar gespritzt und dann bei 20–24 °C und 45–60% relativer Luftfeuchtigkeit gehalten. 15 Tage nach der Behandlung wird der Versuch ausgewertet und das Ergebnis wie im Vorauflauf-Versuch nach derselben Notenskala ausgewertet. Die Resultate sind wie folgt:

Tabelle 4
Post-emergente Wirkung

Aufwandmenge: 4 kg Wirksubstanz/Hektar

| Verb. Nr. | Avena | Setaria | Lolium | Solanum | Sinapis | Stellaria | Phaseolus |
|---|---|---|---|---|---|---|---|
| 1.1 | 1 | 1 | 2 | 1 | 1 | 1 | 2 |
| 1.2 | 1 | 1 | 1 | 1 | 1 | 1 | 2 |
| 1.3 | 1 | 4 | 2 | 1 | 1 | 1 | 1 |
| 1.4 | 1 | 3 | 1 | 1 | 1 | 1 | 3 |
| 1.5 | 1 | 1 | 2 | 1 | 1 | 1 | 2 |
| 1.6 | 1 | 1 | 1 | 1 | 1 | 1 | 2 |
| 1.7 | 1 | 3 | 1 | 1 | 1 | 1 | 3 |
| 1.8 | 1 | 1 | 1 | 1 | 1 | 1 | 2 |
| 1.9 | 1 | 1 | 1 | 1 | 1 | 1 | 2 |
| 1.11 | 1 | 1 | 1 | 1 | 1 | 1 | 3 |
| 1.12 | 1 | 1 | 2 | 1 | 1 | 1 | 8 |
| 1.13 | 1 | 2 | 1 | 1 | 1 | 1 | 2 |
| 1.14 | 2 | 2 | 2 | 1 | 1 | 1 | 2 |
| 1.15 | 1 | 1 | 1 | 1 | 1 | 1 | 7 |
| 1.16 | 2 | 4 | 3 | 1 | 1 | 2 | 9 |
| 1.17 | 1 | 1 | 2 | 1 | 1 | 1 | 2 |
| 1.19 | 1 | 5 | 3 | 1 | 1 | 1 | 8 |
| 1.20 | 1 | 1 | 2 | 1 | 1 | 1 | 1 |
| 1.21 | 1 | 1 | 1 | 1 | 1 | 1 | 1 |
| 1.22 | 1 | 1 | 1 | 1 | 1 | 2 | 4 |
| 1.24 | 1 | 1 | 1 | 1 | 1 | 1 | 2 |
| 1.25 | 1 | 1 | 1 | 1 | 1 | 1 | 2 |
| 1.36 | 2 | 1 | 2 | 1 | 1 | 1 | 8 |
| 1.37 | 1 | 1 | 1 | 1 | 1 | 1 | 3 |
| 1.38 | 1 | 1 | 1 | 1 | 1 | 1 | 3 |
| 1.39 | 1 | 1 | 1 | 1 | 1 | 1 | 3 |
| 1.40 | 1 | 1 | 1 | 1 | 1 | 1 | 2 |
| 1.41 | 1 | 2 | 3 | 1 | 1 | 1 | 6 |
| 1.42 | 1 | 1 | 2 | 1 | 1 | 1 | 1 |
| 1.43 | 1 | 5 | 4 | 1 | 1 | 1 | 5 |
| 1.44 | 1 | 1 | 1 | 1 | 1 | 1 | 2 |
| 1.45 | 1 | 7 | 4 | 1 | 1 | 5 | 3 |
| 1.46 | 1 | 1 | 1 | 1 | 1 | 1 | 1 |
| 1.47 | 1 | 1 | 1 | 1 | 1 | 1 | 1 |
| 1.48 | 1 | 1 | 1 | 1 | 1 | 1 | 2 |
| 1.49 | 1 | 1 | 1 | 1 | 1 | 1 | 2 |
| 1.50 | 1 | 1 | 1 | 1 | 1 | 1 | 2 |
| 1.51 | 1 | 1 | 1 | 1 | 1 | 1 | 2 |
| 2.1 | 1 | 1 | 1 | 1 | 1 | 1 | 2 |
| 2.2 | 1 | 1 | 1 | 1 | 1 | 1 | 2 |
| 2.3 | 1 | 3 | 1 | 1 | 1 | 1 | 2 |

Tabelle 4 (Fortsetzung)

| Verb. Nr. | Avena | Setaria | Lolium | Solanum | Sinapis | Stellaria | Phaseolus |
|---|---|---|---|---|---|---|---|
| 2.4 | 1 | 2 | 1 | 1 | 1 | 1 | 2 |
| 2.5 | 1 | 1 | 1 | 1 | 1 | 1 | 2 |
| 2.6 | 1 | 7 | 3 | 1 | 1 | 1 | 8 |
| 2.10 | 1 | 1 | 3 | 1 | 1 | 1 | 5 |
| 2.11 | 5 | 9 | 9 | 8 | 2 | 8 | 8 |
| 2.12 | 4 | 9 | 7 | 1 | 2 | 5 | 5 |
| 2.13 | 1 | 1 | 2 | 1 | 1 | 2 | 1 |
| 2.14 | 4 | 6 | 2 | 1 | 2 | 7 | 3 |
| 2.16 | 4 | 1 | 3 | 1 | 2 | 4 | 3 |
| 2.17 | 4 | 1 | 5 | 2 | 2 | 5 | 3 |
| 2.18 | 1 | 1 | 2 | 1 | 1 | 1 | 1 |
| 2.19 | 6 | 8 | 7 | 8 | 1 | 4 | 4 |
| 2.20 | 1 | 1 | 1 | 1 | 1 | 1 | 2 |
| 2.21 | 1 | 1 | 1 | 1 | 1 | 1 | 2 |
| 2.22 | 1 | 1 | 1 | 1 | 1 | 1 | 1 |
| 2.23 | 5 | 9 | 2 | 1 | 2 | 4 | 9 |

Beispiel B3: Wirkung als Reisherbizide

Die Unkräuter Echinochloa crus galli und Monochoria vaginalis werden in mit Erde beschickte Plastikbecher (Oberfläche 60 $cm^2$, Volumen 500 ml) ausgesät. Nach der Saat werden die Becher mit Wasser aufgefüllt, bis das Wasser die Erdoberfläche erreicht. 3 Tage nach der Saat wird das Wasser so weit nachgefüllt, dass sich der Wasserspiegel etwas oberhalb (3 bis 5 mm) der Erdoberfläche befindet. Die Testsubstanzen werden 3 Tage nach der Saat als wässrige Emulsion auf die Wasseroberfläche der Becher gespritzt. Die eingesetzte Dosis entspricht einer Wirkstoffmenge von 4 kg/ha und einer Menge an Spritzbrühe von 550 l/ha. Nach der Applikation werden die Becher im Gewächshaus bei Bedingungen, welche für das Wachstum der Unkräuter optimal sind, d.h., bei 25–30 °C und hoher Luftfeuchtigkeit, gehalten. Die Versuche werden je nach Wachstumsgeschwindigkeit und Pflanzenart 2–3 Wochen nach der Applikation ausgewertet. Die Bewertung erfolgt nach einer linearen Notenskala von 1 bis 9, wobei folgende Kriterien gelten:

1 Pflanze nicht gekeimt
2–3 Zwischenstufen
5 mittlere Wirkung
6–8 Zwischenstufen
9 keine Wirkung

Tabelle 5

| Verbindung Nr. | Echinochloa c.g. | Monochoria vag. |
|---|---|---|
| 1.24 | 1 | 3 |
| 1.25 | 1 | 1 |
| 1.45 | 6 | 7 |
| 1.46 | 3 | 5 |
| 1.47 | 1 | 1 |
| 1.48 | 4 | 6 |
| 1.49 | 2 | 2 |
| 1.50 | 2 | 5 |
| 2.12 | 3 | 4 |
| 2.13 | 2 | 1 |
| 2.14 | 3 | 1 |
| 2.15 | 5 | 3 |
| 2.16 | 3 | 1 |
| 2.17 | 3 | 1 |
| 2.18 | 1 | 1 |
| 2.19 | 2 | 2 |
| 2.20 | 1 | 1 |
| 2.21 | 1 | 2 |
| 2.22 | 1 | 1 |

Beispiel B4:

Wirkung gegen Nilaparvata lugens (Nymphen)

Der Test wird an wachsenden Pflanzen durchgeführt. Es werden jeweils 10 bis 15 Reispflanzen (ca. 20 Tage alt, Höhe ca. 15 cm) in Töpfe mit einem Durchmesser von 5,5 cm eingepflanzt. Die

Pflanzen werden auf einem Drehteller mit jeweils 40 ml einer acetonischen Lösung, welche 400 ppm des zu prüfenden Wirkstoffs enthält, besprüht. Nach dem Antrocknen des Spritzbelages erfolgt die Besiedlung jeder Pflanze mit je 20 Nymphen der Spezies Nilaparvata lugens (zweites bis drittes Stadium). Um ein Entweichen der Zikaden zu verhindern, wird über die besiedelten Pflanzen jeweils ein Plexiglaszylinder gestülpt und dieser mit einem Gazedeckel abgedeckt. Der Versuch wird bei einer Temperatur von 24–28 °C, 55–65% relativer Luftfeuchtigkeit und 16-stündiger Beleuchtung durchgeführt. Die Auswertung auf % Mortalität erfolgt nach 6 Tagen.

Verbindungen der Formeln (I) und (III) zeigen in diesem Test eine gute Wirkung. So wird beispielsweise mit den Verbindungen Nr. 1.51 und 2.6 eine Mortalität von 80–100% erzielt.

Beispiel B5:
Wirkung gegen Bodeninsekten
(Diabrotica balteata)

Maiskeimlinge (Grösse 1–3 cm), welche sich in einem mit einer Filterrondelle ausgestatteten Plastikbecher (Volumen 200 ml) befinden, werden mit 1 ml einer 400 ppm Wirkstoff enthaltenden Testlösung beträufelt und anschliessend mit jeweils 10 Larven von Diabrotica balteata besetzt. Der Versuch wird bei einer Temperatur von 22–26 °C und 40–60% relativer Luftfeuchtigkeit bei Tageslicht durchgeführt. Nach 6 Tagen erfolgt die Auswertung. Verbindungen der Formeln (I) und (III) zeigen in diesem Test eine gute Wirkung. Beispielsweise wird mit den Verbindungen Nr. 1.13 und 2.14 eine Mortalität von über 80% erreicht.

Beispiel B6:
Systemisch-insektizide Wirkung gegen Aphis craccivora

Erbsenkeimlinge werden 24 Stunden nach Infestierung mit Blattläusen der Spezies Aphis craccivora kurz in 20 ml einer wässrigen Brühe getaucht, welche 400 ppm des zu prüfenden Wirkstoffs enthält. Die wässrige Brühe wird aus einem Emulsionskonzentrat oder einer benetzbaren Pulverzubereitung des betreffenden Wirkstoffs hergestellt. Die Keimlinge werden anschliessend in ein kleines Glas (Volumen 20 ml) mit der Testlösung gestellt. Der Versuch wird bei einer Temperatur von 19–23 °C und 45–65% relativer Luftfeuchtigkeit unter Tageslicht durchgeführt. Die Auswertung erfolgt nach 3 und 5 Tagen. Die Verbindungen der Formeln (I) und (III) zeigen in diesem Test eine gute Wirkung. Beispielsweise wird mit den Verbindungen Nr. 1.10, 2.2 und 2.23 eine Mortalität von über 80% erzielt.

**Patentansprüche für Vertragsstaaten BE, CH, DE, FR, GB, IT, LI, NL, SE**

1. 4-Methylamino-1,2,4-triazin-5-one der Formel (I)

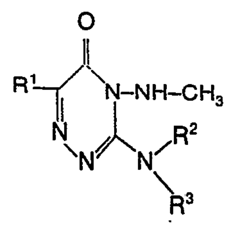

I

worin

$R^1$ für $C_3$–$C_8$-Cycloalkyl, durch $C_1$–$C_4$-Alkyl substituiertes $C_3$–$C_8$-Cycloalkyl, Halogen-$C_3$–$C_8$-cycloalkyl, $C_3$–$C_8$-Alkyl, Halogen-$C_3$–$C_8$-alkyl, 2-Furyl, 2-Thienyl, Phenyl, Benzyl oder durch Substituenten aus der Gruppe Halogen, $C_1$–$C_4$-Alkyl, Halogen-$C_1$–$C_4$-alkyl, $C_1$–$C_4$-Alkoxy oder Halogen-$C_1$–$C_4$-alkoxy substituiertes Phenyl oder Benzyl,

$R^2$ für Wasserstoff oder Methyl und

$R^3$ für Cyclopropyl, Cyclopropylmethyl, $C_1$–$C_4$-Alkyl, $C_3$–$C_4$-Alkenyl oder $C_3$–$C_4$-Alkinyl

stehen, wobei nicht gleichzeitig $R^1$ für tert.Butyl, $R^2$ für Wasserstoff und $R^3$ für Methyl stehen dürfen.

2. Verbindungen gemäss Anspruch 1, dadurch gekennzeichnet, dass $R^1$ eine verzweigte $C_3$–$C_6$-Alkyl- oder eine verzweigte Halogen-$C_3$–$C_6$-alkylgruppe bedeutet.

3. Verbindungen gemäss Anspruch 1, dadurch gekennzeichnet, dass $R^1$ Phenyl oder durch Substituenten aus der Gruppe Halogen, $C_1$–$C_4$-Alkyl, Halogen-$C_1$–$C_4$-alkyl, $C_1$–$C_4$-Alkoxy oder Halogen-$C_1$–$C_4$-alkoxy substituiertes Phenyl bedeutet.

4. Verbindungen gemäss Anspruch 2, dadurch gekennzeichnet, dass $R^1$ verzweigtes $C_3$–$C_6$-Alkyl bedeutet.

5. Verbindungen gemäss Anspruch 1, dadurch gekennzeichnet, dass $R^2$ Wasserstoff bedeutet.

6. Verbindungen gemäss Anspruch 2, dadurch gekennzeichnet, dass $R^3$ für $C_1$–$C_4$-Alkyl steht.

7. Verbindungen gemäss Anspruch 6, dadurch gekennzeichnet, dass $R^3$ für Methyl steht.

8. Verbindungen gemäss Anspruch 1, dadurch gekennzeichnet, dass $R^1$ für verzweigtes $C_3$–$C_6$-Alkyl oder verzweigtes Halogen-$C_3$–$C_6$-alkyl, $R^2$ für Wasserstoff oder Methyl und $R^3$ für $C_1$–$C_4$-Alkyl stehen.

9. Verbindungen gemäss Anspruch 1, dadurch gekennzeichnet, dass $R^1$ für verzweigtes $C_3$–$C_6$-Alkyl, $R^2$ für Wasserstoff und $R^3$ für Methyl stehen.

10. Eine Verbindung gemäss Anspruch 1, ausgewählt aus

3,4-Bis-(methylamino)-6-isopropyl-4H-1,2,4-triazin-5-on,

3-Dimethylamino-4-methylamino-6-(1,1-dimethyläthyl)-4H-1,2,4-triazin-5-on,

3,4-Bis-(methylamino)-6-(1-methylpropyl)-4H-1,2,4-triazin-5-on,

3,4-Bis-(methylamino)-6-phenyl-4H-1,2,4-triazin-5-on und

3,4-Bis-(methylamino)-6-(2-fluorphenyl)-4H-1,2,4-triazin-5-on.

11. Verbindungen der Formel (III)

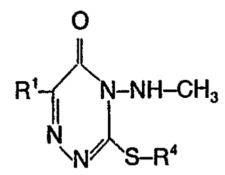

III

worin $R^1$ die unter Formel (I) im Anspruch 1 gegebene Bedeutung hat und $R^4$ für Methyl oder Äthyl steht, wobei nicht gleichzeitig $R^1$ für tert.Butyl und $R^4$ für Methyl stehen dürfen.

12. Verbindungen der Formel (VII)

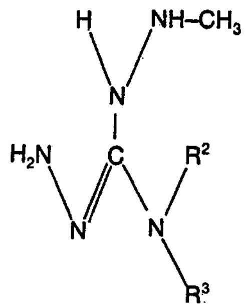

VII

worin $R^2$ und $R^3$ die unter Formel (I) im Anspruch 1 gegebenen Bedeutungen haben, oder ein Säureadditionssalz dieser Verbindungen.

13. Verfahren zur Herstellung der Verbindungen der Formel (I) gemäss Anspruch 1, dadurch gekennzeichnet, dass man entweder

a) ein 4-Amino-4H-1,2,4-triazin-5-on der Formel (II)

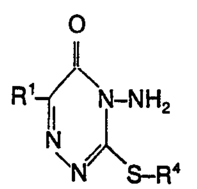

II

worin $R^1$ die unter Formel (I) im Anspruch 1 gegebene Bedeutung hat und $R^4$ für Methyl oder Äthyl steht, mit einem Methylierungsmittel in ein 4-Methylamino-4H-1,2,4-triazin-5-on der Formel (III)

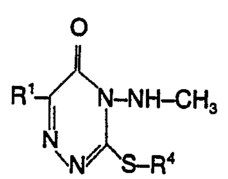

III

überführt und dieses bei erhöhter Temperatur mit einem Amin der Formel (IV)

$$H{-}NR^2R^3 \qquad IV$$

worin $R^2$ und $R^3$ die unter Formel (I) im Anspruch 1 gegebenen Bedeutungen haben, umsetzt; oder

b) ein 3,4-Diamino-4H-1,2,4-triazin-5-on der Formel (V)

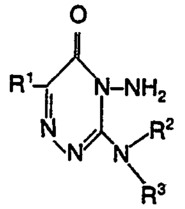

V

worin $R^1$, $R^2$ und $R^3$ die unter Formel (I) im Anspruch 1 gegebenen Bedeutungen haben, mit einem Methylierungsmittel umsetzt.

14. Verfahren zur Herstellung der Verbindungen der Formel (I) gemäss Anspruch 1, dadurch gekennzeichnet, dass man ein α-Ketocarbonsäurederivat der Formel (VI)

$$R^1{-}CO{-}A \qquad VI$$

worin

$R^1$ die in Anspruch 1 angegebene Bedeutung hat und A für $-COOH$, $-COO-C_1-C_4$-Alkyl, $-COS-C_1-C_4$-Alkyl, $-CONH_2$, $-CONH-C_1-C_4$-Alkyl oder $-CONH-CO-C_1-C_4$-Alkyl steht, mit einem Guanidinderivat der Formel (VII)

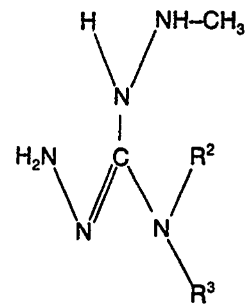

VII

worin

$R^2$ und $R^3$ die in Anspruch 1 angegebene Bedeutung haben, kondensiert.

15. Mittel zur Bekämpfung von unerwünschtem Pflanzenwuchs und von schädlichen Insekten, dadurch gekennzeichnet, dass es neben Träger- und/oder anderen Zusatzstoffen als aktive Komponente eine Verbindung der Formel (I) gemäss Anspruch 1 oder (III) gemäss Anspruch 11 enthält.

16. Mittel nach Anspruch 15, dadurch gekennzeichnet, dass es 0,1 bis 99 Gewichtsprozent Wirkstoff der Formel (I) oder (III), 99,9 bis 1 Gewichtsprozent an einem oder mehreren Zusatzstoffen und 0 bis 25 Gewichtsprozent eines Tensides enthält.

17. Verwendung eines Wirkstoffs gemäss Anspruch 1 zur Bekämpfung schädlicher Insekten.

18. Verwendung eines Wirkstoffes gemäss Anspruch 1 zur Bekämpfung von unerwünschtem Pflanzenwuchs.

19. Verwendung nach Anspruch 18 zur Bekämpfung von Unkräutern in Kulturpflanzenbeständen, vorzugsweise in Kulturen von Getreide, Mais, Soja und Zuckerrohr.

20. Verwendung nach Anspruch 19 zur Bekämpfung von Unkräutern in Kulturen von Zuckerrohr.

21. Verfahren zur Bekämpfung von Unkräutern in Kulturpflanzenbeständen, dadurch gekennzeichnet, dass man eine wirksame Menge eines Wirkstoffes gemäss Anspruch 1 oder eines Mittels, welches eine dieser Verbindungen enthält, auf die Kulturen appliziert.

**Patentansprüche für den Vertragsstaat AT**

1. Verfahren zur Herstellung von 4-Methylamino-1,2,4-triazin-5-onen der Formel (I)

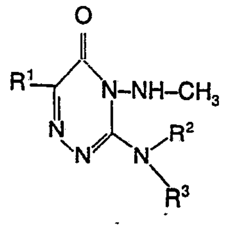

I

worin

$R^1$ für $C_3$–$C_8$-Cycloalkyl, durch $C_1$–$C_4$-Alkyl substituiertes $C_3$–$C_8$-Cycloalkyl, Halogen-$C_3$–$C_8$-cycloalkyl, $C_3$–$C_8$-Alkyl, Halogen-$C_3$–$C_8$-alkyl, 2-Furyl, 2-Thienyl, Phenyl, Benzyl oder durch Substituenten aus der Gruppe Halogen, $C_1$–$C_4$-Alkyl, Halogen-$C_1$–$C_4$-alkyl, $C_1$–$C_4$-Alkoxy oder Halogen-$C_1$–$C_4$-alkoxy substituiertes Phenyl oder Benzyl,

$R^2$ für Wasserstoff oder Methyl und

$R^3$ für Cyclopropyl, Cyclopropylmethyl, $C_1$–$C_4$-Alkyl, $C_3$–$C_4$-Alkenyl oder $C_3$–$C_4$-Alkinyl stehen, wobei nicht gleichzeitig $R^1$ für tert.Butyl, $R^2$ für Wasserstoff und $R^3$ für Methyl stehen dürfen, dadurch gekennzeichnet, dass man entweder

a) ein 4-Amino-4H-1,2,4-triazin-5-on der Formel (II)

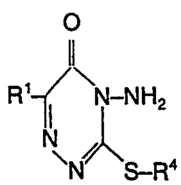

II

worin $R^1$ die unter Formel (I) angegebene Bedeutung hat und $R^4$ für Methyl oder Äthyl steht, mit einem Methylierungsmittel in ein 4-Methylamino-4H-1,2,4-triazin-5-on der Formel (III)

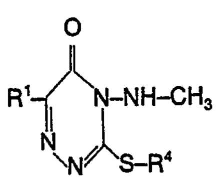

III

überführt und dieses bei erhöhter Temperatur mit einem Amin der Formel (IV)

$$H–NR^2R^3 \qquad IV$$

worin $R^2$ und $R^3$ die unter Formel (I) angegebenen Bedeutungen haben, umsetzt; oder

b) ein 3,4-Diamino-4H-1,2,4-triazin-5-on der Formel (V)

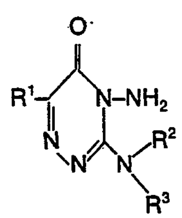

V

worin $R^1$, $R^2$ und $R^3$ die unter Formel (I) angegebenen Bedeutungen haben, mit einem Methylierungsmittel umsetzt; oder

c) ein α-Ketocarbonsäurederivat der Formel (VI)

$$R^1–CO–A \qquad VI$$

worin $R^1$ die unter Formel (I) angegebene Bedeutung hat, mit einem Guanidinderivat der Formel (VII)

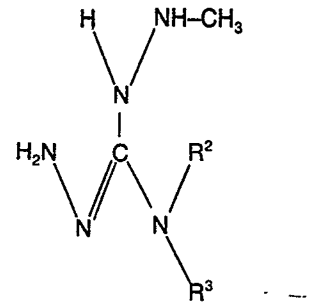

VII

worin

$R^2$ für Wasserstoff oder Methyl und

$R^3$ für Cyclopropyl, Cyclopropylmethyl, $C_1$–$C_4$-Alkyl, $C_3$–$C_4$-Alkenyl oder $C_3$–$C_4$-Alkinyl stehen, kondensiert.

2. Verfahren gemäss Anspruch 1, dadurch gekennzeichnet, dass $R^1$ eine verzweigte $C_3$–$C_6$-Alkyl- oder eine verzweigte Halogen-$C_3$–$C_6$-alkylgruppe bedeutet.

3. Verfahren gemäss Anspruch 1, dadurch gekennzeichnet, dass $R^1$ Phenyl oder durch Substituenten aus der Gruppe Halogen, $C_1$–$C_4$-Alkyl, Halogen-$C_1$–$C_4$-alkyl, $C_1$–$C_4$-Alkoxy oder Halogen-$C_1$–$C_4$-alkoxy substituiertes Phenyl bedeutet.

4. Verfahren gemäss Anspruch 2, dadurch gekennzeichnet, dass $R^1$ verzweigtes $C_3$–$C_6$-Alkyl bedeutet.

5. Verfahren gemäss Anspruch 1, dadurch gekennzeichnet, dass $R^2$ Wasserstoff bedeutet.

6. Verfahren gemäss Anspruch 2, dadurch gekennzeichnet, dass $R^3$ für $C_1$–$C_4$-Alkyl steht.

7. Verfahren gemäss Anspruch 6, dadurch gekennzeichnet, dass $R^3$ für Methyl steht.

8. Verfahren gemäss Anspruch 1, dadurch gekennzeichnet, dass $R^1$ für verzweigtes $C_3$–$C_6$-Alkyl oder verzweigtes Halogen-$C_3$–$C_6$-alkyl, $R^2$ für Wasserstoff oder Methyl und $R^3$ für $C_1$–$C_4$-Alkyl stehen.

9. Verfahren gemäss Anspruch 1, dadurch gekennzeichnet, dass $R^1$ für verzweigtes $C_3$–$C_6$-Alkyl, $R^2$ für Wasserstoff und $R^3$ für Methyl stehen.

10. Verfahren gemäss Anspruch 1, dadurch gekennzeichnet, dass man

3,4-Bis-(methylamino)-6-isopropyl-4H-1,2,4-triazin-5-on,

3-Dimethylamino-4-methylamino-6-(1,1-dimethyläthyl)-4H-1,2,4-triazin-5-on,

3,4-Bis-(methylamino)-6-(1-methylpropyl)-4H-1,2,4-triazin-5-on,

3,4-Bis-(methylamino)-6-phenyl-4H-1,2,4-triazin-5-on oder

3,4-Bis-(methylamino)-6-(2-fluorphenyl)-4H-1,2,4-triazin-5-on

herstellt.

11. Mittel zur Bekämpfung von unerwünschtem Pflanzenwuchs und von schädlichen Insekten, dadurch gekennzeichnet, dass es neben Träger-und/oder anderen Zusatzstoffen als aktive Komponente eine Verbindung der Formel (I) oder (III) wie in Anspruch 1 definiert enthält.

12. Mittel nach Anspruch 11, dadurch gekennzeichnet, dass es 0,1 bis 99 Gewichtsprozent Wirkstoff der Formel (I) oder (III) gemäss Anspruch 1, 99,9 bis 1 Gewichtsprozent an einem oder mehreren Zusatzstoffen und 0 bis 25 Gewichtsprozent eines Tensides enthält.

13. Verwendung eines Wirkstoffs der Formel (I) gemäss Anspruch 1 zur Bekämpfung schädlicher Insekten.

14. Verwendung eines Wirkstoffes der Formel (I) gemäss Anspruch 1 zur Bekämpfung von unerwünschtem Pflanzenwuchs.

15. Verwendung nach Anspruch 14 zur Bekämpfung von Unkräutern in Kulturpflanzenbeständen, vorzugsweise in Kulturen von Getreide, Mais, Soja und Zuckerrohr.

16. Verwendung nach Anspruch 15 zur Bekämpfung von Unkräutern in Kulturen von Zuckerrohr.

17. Verfahren zur Bekämpfung von Unkräutern in Kulturpflanzenbeständen, dadurch gekennzeichnet, dass man eine wirksame Menge eines Wirkstoffes der Formel (I) gemäss Anspruch 1 oder eines Mittels, welches eine solche Verbindung enthält, auf die Kulturen appliziert.

**Claims for the Contracting States BE, CH, DE, FR, GB, IT, LI, NL, SE**

1. 4-methylamino-1,2,4-triazin-5-ones of formula (I)

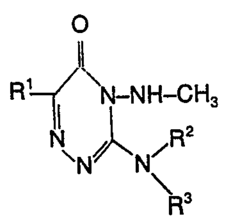

I

wherein

$R^1$ is $C_3$–$C_8$cycloalkyl or $C_3$–$C_8$cycloalkyl which is substituted by $C_1$–$C_4$alkyl; $C_3$–$C_8$halocycloalkyl, $C_3$–$C_8$alkyl, $C_3$–$C_8$haloalkyl, 2-furyl, 2-thienyl, phenyl, benzyl, or phenyl or benzyl each substituted by substituents from the group consisting of halogen, $C_1$–$C_4$alkyl, $C_1$–$C_4$haloalkyl, $C_1$–$C_4$alkoxy or $C_1$–$C_4$haloalkoxy,

$R^2$ is hydrogen or methyl, and

$R^3$ is cyclopropyl, cyclopropylmethyl, $C_1$–$C_4$alkyl, $C_3$–$C_4$alkenyl or $C_3$–$C_4$alkynyl,

with the proviso that simultaneously $R^1$ may not be tert-butyl, $R^2$ may not be hydrogen and $R^3$ may not be methyl.

2. Compounds according to claim 1, wherein $R^1$ is a branched $C_3$–$C_6$alkyl group or a branched $C_3$–$C_6$haloalkyl group.

3. Compounds according to claim 1, wherein $R^1$ is phenyl or phenyl which is substituted by substituents from the group consisting of halogen, $C_1$–$C_4$alkyl, $C_1$–$C_4$haloalkyl, $C_1$–$C_4$alkoxy or $C_1$–$C_4$haloalkoxy.

4. Compounds according to claim 2, wherein $R^1$ is branched $C_3$–$C_6$alkyl.

5. Compounds according to claim 1, wherein $R^2$ is hydrogen.

6. Compounds according to claim 2, wherein $R^3$ is $C_1$–$C_4$alkyl.

7. Compounds according to claim 6, wherein $R^3$ is methyl.

8. Compounds according to claim 1, wherein $R^1$ is branched $C_3$–$C_6$alkyl or branched $C_3$–$C_6$haloalkyl, $R^2$ is hydrogen or methyl, and $R^3$ is $C_1$–$C_4$alkyl.

9. Compounds according to claim 1, wherein $R^1$ is branched $C_3$–$C_6$alkyl, $R^2$ is hydrogen and $R^3$ is methyl.

10. A compound according to claim 1, selected from
3,4-bis(methylamino)-6-isopropyl-4H-1,2,4-triazin-5-one,
3-dimethylamino-4-methylamino-6-(1,1-dimethylethyl)-4H-1,2,4-triazin-5-one,
3,4-bis(methylamino)-6-(1-methylpropyl)-4H-1,2,4-triazin-5-one,
3,4-bis(methylamino)-6-phenyl-4H-1,2,4-triazin-5-one and
3,4-bis(methylamino)-6-(2-fluorophenyl)-4H-1,2,4-triazin-5-one.

11. Compounds of formula (III)

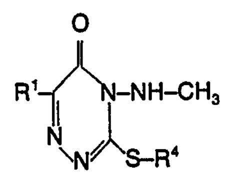

III

wherein $R^1$ is as defined for formula (I) in claim 1 and $R^4$ is methyl or ethyl, with the proviso that simultaneously $R^1$ may not be tert-butyl and $R^4$ may not be methyl.

12. Compounds of formula (VII)

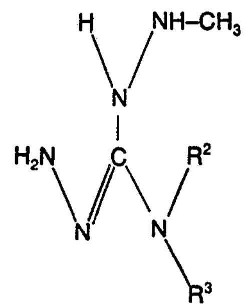

VII

wherein $R^2$ and $R^3$ are as defined for formula (I) in claim 1, or an acid addition salt thereof.

13. A process for the preparation of compounds of formula (I) according to claim 1, which comprises either

a) converting a 4-amino-4H-1,2,4-triazin-5-one of formula (II)

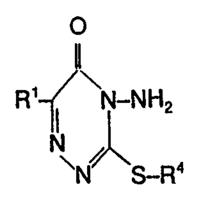

II

wherein $R^1$ is as defined for formula (I) in claim 1 and $R^4$ is methyl or ethyl, with a methylating agent, into a 4-methylamino-4H-1,2,4-triazin-5-one of formula (III)

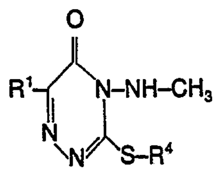

III

and reacting said compound of formula (III), at elevated temperature, with an amine of formula (IV)

$$H{-}NR^2R^3 \qquad IV$$

wherein $R^2$ and $R^3$ are as defined for formula (I) in claim 1; or

b) reacting a 3,4-diamino-4H-1,2,4-triazin-5-one of formula (V)

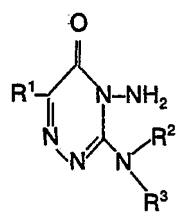

V

wherein $R^1$, $R^2$ and $R^3$ are as defined for formula (I) in claim 1, with a methylating agent.

14. A process for the preparation of compounds of formula (I) according to claim 1, which comprises condensing an α-ketocarboxylic acid derivative of formula (VI)

$$R^1{-}CO{-}A \qquad VI$$

wherein $R^1$ is as defined in claim 1 and A is $-COOH$, $-COO-C_1-C_4$alkyl, $-COS-C_1-C_4$alkyl, $-CONH_2$, $-CONH-C_1-C_4$alkyl or $-CONH-CO-C_1-C_4$ alkyl, with a guanidine derivative of formula (VII)

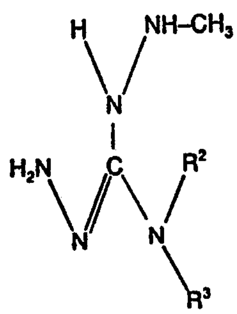

VII

wherein $R^2$ and $R^3$ are as defined in claim 1.

15. A composition for controlling undesirable plant growth and harmful insects, which composition contains, as active component, a compound of formula (I) according to claim 1 or (III) according to claim 11, together with carriers and/or other adjuvants.

16. A composition according to claim 15, which contains 0.1 to 99 percent by weight of a compound of formula (I) or (III), 99.9 to 1 percent by weight of one or more adjuvants, and 0 to 25 percent by weight of a surfactant.

17. The use of a compound according to claim 1 for controlling harmful insects.

18. The use of a compound according to claim 1 for controlling undesirable plant growth.

19. The use according to claim 18 for controlling weeds in crops of cultivated plants, preferably in crops of cereals, maize, soybeans and sugar cane.

20. The use according to claim 19 for controlling weeds in crops of sugar cane.

21. A method of controlling weeds in crops of cultivated plants, which comprises applying to said crops an effective amount of a compound according to claim 1 or of a composition containing such a compound.

**Claims for the Contracting State AT**

1. A process for the preparation of 4-methylamino-1,2,4-triazin-5-ones of formula (I)

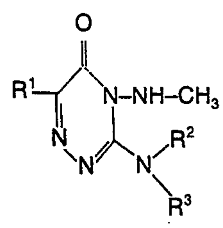

I

wherein

$R^1$ is $C_3-C_8$cycloalkyl or $C_3-C_8$cycloalkyl which is substituted by $C_1-C_4$alkyl; $C_3-C_8$halocycloalkyl, $C_3-C_8$alkyl, $C_3-C_8$haloalkyl, 2-furyl, 2-thienyl, phenyl, benzyl, or phenyl or benzyl each substituted by substituents from the group consisting of halogen, $C_1-C_4$alkyl, $C_1-C_4$haloalkyl, $C_1-C_4$alkoxy or $C_1-C_4$haloalkoxy,

$R^2$ is hydrogen or methyl, and

$R^3$ is cyclopropyl, cyclopropylmethyl, $C_1-C_4$alkyl, $C_3-C_4$alkenyl or $C_3-C_4$alkynyl,

with the proviso that simultaneously $R^1$ may not be tert-butyl, $R^2$ may not be hydrogen and $R^3$ may not be methyl, which comprises either

a) converting a 4-amino-4H-1,2,4-triazin-5-one of formula (II)

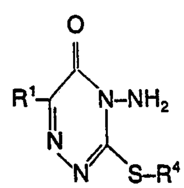

II

wherein $R^1$ is as defined for formula (I) and $R^4$ is methyl or ethyl, with a methylating agent, into a 4-methylamino-4H-1,2,4-triazin-5-one of formula (III)

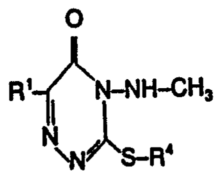

III

and reacting said compound of formula (III), at elevated temperature, with an amine of formula (IV)

$$H-NR^2R^3 \qquad IV$$

wherein $R^2$ and $R^3$ are as defined for formula (I); or

b) reacting a 3,4-diamino-4H-1,2,4-triazin-5-one of formula (V)

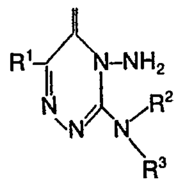

V

wherein $R^1$, $R^2$ and $R^3$ are as defined for formula (I), with a methylating agent; or

c) condensing an α-ketocarboxylic acid derivative of formula (VI)

$$R^1-CO-A \qquad VI$$

wherein $R^1$ is as defined for formula (I), with a guanidine derivative of formula (VII)

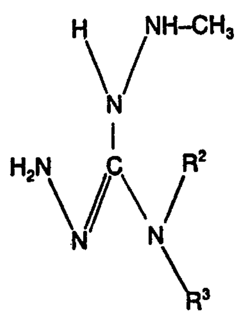

VII

wherein

$R^2$ is hydrogen or methyl, and

$R^3$ is cyclopropyl, cyclopropylmethyl, $C_1$–$C_4$alkyl, $C_3$–$C_4$alkenyl or $C_3$–$C_4$alkynyl.

2. A process according to claim 1, wherein $R^1$ is a branched $C_3$–$C_6$alkyl group or a branched $C_3$–$C_6$haloalkyl group.

3. A process according to claim 1, wherein $R^1$ is phenyl or phenyl which is substituted by substituents from the group consisting of halogen, $C_1$–$C_4$alkyl, $C_1$–$C_4$haloalkyl, $C_1$–$C_4$alkoxy or $C_1$–$C_4$haloalkoxy.

4. A process according to claim 2, wherein $R^1$ is branched $C_3$–$C_6$alkyl.

5. A process according to claim 1, wherein $R^2$ is hydrogen.

6. A process according to claim 2, wherein $R^3$ is $C_1$–$C_4$alkyl.

7. A process according to claim 6, wherein $R^3$ is methyl.

8. A process according to claim 1, wherein $R^1$ is branched $C_3$–$C_6$alkyl or branched $C_3$–$C_6$haloalkyl, $R^2$ is hydrogen or methyl, and $R^3$ is $C_1$–$C_4$alkyl.

9. A process according to claim 1, wherein $R^1$ is branched $C_3$–$C_6$alkyl, $R^2$ is hydrogen and $R^3$ is methyl.

10. A process according to claim 1, wherein
3,4-bis(methylamino)-6-isopropyl-4H-1,2,4-triazin-5-one,
3-dimethylamino-4-methylamino-6-(1,1-dimethylethyl)-4H-1,2,4-triazin-5-one,
3,4-bis(methylamino)-6-(1-methylpropyl)-4H-1,2,4-triazin-5-one,
3,4-bis(methylamino)-6-phenyl-4H-1,2,4-triazin-5-one or
3,4-bis(methylamino)-6-(2-fluorophenyl)-4H-1,2,4-triazin-5-one
is prepared.

11. A composition for controlling undesirable plant growth and harmful insects, which composition contains, as active component, a compound of formula (I) or (III) as defined in claim 1, together with carriers and/or other adjuvants.

12. A composition according to claim 11, which contains 0.1 to 99 percent by weight of a compound of formula (I) or (III) according to claim 1, 99.9 to 1 percent by weight of one or more adjuvants, and 0 to 25 percent by weight of a surfactant.

13. The use of a compound of formula (I) according to claim 1 for controlling harmful insects.

14. The use of a compound of formula (I) according to claim 1 for controlling undesirable plant growth.

15. The use according to claim 14 for controlling weeds in crops of cultivated plants, preferably in crops of cereals, maize, soybeans and sugar cane.

16. The use according to claim 15 for controlling weeds in crops of sugar cane.

17. A method of controlling weeds in crops of cultivated plants, which comprises applying to said crops an effective amount of a compound of formula (I) according to claim 1 or of a composition containing such a compound.

**Revendications pour les Etats contractants BE, CH, DE, FR, GB, IT, LI, NL, SE**

1. 4-méthylamino-1,2,4-triazine-5-ones de formule I

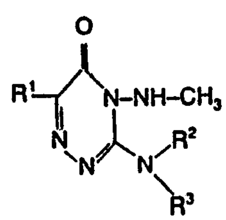

I

dans laquelle

$R^1$ représente un groupe cycloalkyle en C 3–C 8, un groupe cycloalkyle en C 3–C 8 substitué par un groupe alkyle en C 1–C 4, un groupe halogénocycloalkyle en C 3–C 8, alkyle en C 3–C 8, halogénoalkyle en C 3–C 8, 2-furyle, 2-thiényle, phényle, benzyle ou phényle ou benzyle portant des substituants pris parmi les halogènes, les groupes alkyle en C 1–C 4, halogénoalkyle en C 1–C 4, alcoxy en C 1–C 4 ou halogénoalcoxy en C 1–C 4,

$R^2$ représente l'hydrogène ou un groupe méthyle, et

$R^3$ représente un groupe cyclopropyle, cyclopropylméthyle, alkyle en C 1–C 4, alcényle en C 3–C 4 ou alcynyle en C 3–C 4,

étant spécifié que $R^1$, $R^2$ et $R^3$ ne peuvent représenter simultanément: $R^1$ un groupe tert-butyle, $R^2$ l'hydrogène et $R^3$ un groupe méthyle.

2. Composés selon la revendication 1, caractérisés en ce que $R^1$ représente un groupe alkyle ramifié en C 3–C 6 ou un groupe halogénoalkyle ramifié en C 3–C 6.

3. Composés selon la revendication 1, caractérisés en ce que $R^1$ représente un groupe phényle ou un groupe phényle portant des substituants pris parmi les halogènes, les groupes alkyle en C 1–C 4, halogénoalkyle en C 1–C 4, alcoxy en C 1–C 4 ou halogénoalcoxy en C 1–C 4.

4. Composés selon la revendication 2, caractérisés en ce que $R^1$ représente un groupe alkyle ramifié en C 3–C 6.

5. Composés selon la revendication 1, caractérisés en ce que $R^2$ représente l'hydrogène.

6. Composés selon la revendication 2, caractérisés en ce que $R^3$ représente un groupe alkyle en C 1–C 4.

7. Composés selon la revendication 6, caractérisés en ce que $R^3$ représente un groupe méthyle.

8. Composés selon la revendication 1, caractérisés en ce que $R^1$ représente un groupe alkyle ramifié en C 3–C 6 ou un groupe halogénoalkyle ramifié en C 3–C 6, $R^2$ l'hydrogène ou un groupe méthyle et $R^3$ un groupe alkyle en C 1–C 4.

9. Composés selon la revendication 1, caractérisés en ce que $R^1$ représente un groupe alkyle ramifié en C 3–C 6, $R^2$ l'hydrogène et $R^3$ un groupe méthyle.

10. Un composé selon la revendication 1, choisi parmi les suivants:

3,4-bis-(méthylamino)-6-isopropyl-4H-1,2,4-triazine-5-one,

3-diméthylamino-4-méthylamino-6-(1,1-diméthyléthyl)-4H-1,2,4-triazine-5-one,

3,4-bis-(méthylamino)-6-(1-méthylpropyl)-4H-1,2,4-triazine-5-one,

3,4-bis-(méthylamino)-6-phényl-4H-1,2,4-triazine-5-one, et

3,4-bis-(méthylamino)-6-(2-fluorophényl)-4H-1,2,4-triazine-5-one.

11. Composés de formule III

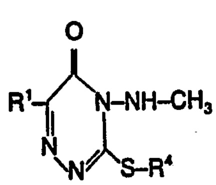

III

dans laquelle $R^1$ a les significations indiquées dans la revendication 1 en reférence à la formule I et $R^4$ représente un groupe méthyle ou éthyle, étant spécifié que $R^1$ et $R^4$ ne peuvent représenter simultanément: $R^1$ un groupe tert-butyle et $R^4$ un groupe méthyle.

12. Composés de formule VII

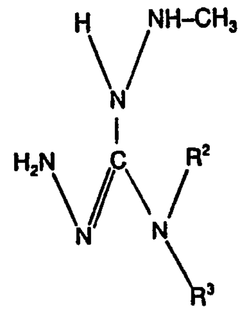

VII

dans laquelle $R^2$ et $R^3$ ont les significations indiquées dans la revendication 1 en référence à la formule I, ou un sel de ces composés formés par addition avec un acide.

13. Procédé de préparation des composés de formule I selon la revendication 1, caractérisé en ce que:

a) on convertit une 4-amino-4H-1,2,4-triazine-5-one de formule II

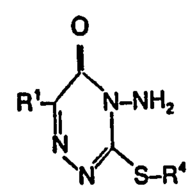

II

dans laquelle $R^1$ a les significations indiquées dans la revendication 1 en référence à la formule (I) et $R^4$ représente un groupe méthyle ou éthyle, à l'aide d'un agent méthylant, en une 4-méthylamino-4H-1,2,4-triazine-5-one de formule III

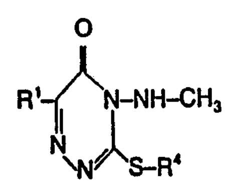

III

qu'on fait réagir à température élevée avec une amine de formule IV

$$H\text{–}NR^2R^3 \qquad IV$$

dans laquelle $R^2$ et $R^3$ ont les significations indiquées dans la revendication 1 en référence à la formule I; ou bien

b) on fait réagir une 3,4-diamino-4H-1,2,4-triazine-5-one de formule V

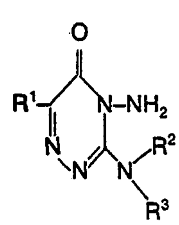

V

dans laquelle $R^1$, $R^2$ et $R^3$ ont les significations indiquées dans la revendication 1 en référence à la formule I, avec un agent méthylant.

14. Procédé de préparation des composés de formule I selon la revendication 1, caractérisé en ce que l'on condense un dérivé d'acide α-cétocarboxylique de formule VI

$$R^1\text{–CO–A} \qquad \text{VI}$$

dans laquelle
$R^1$ a les significations indiquées dans la revendication 1 et A représente un groupe –COOH, –COO–alkyle en C 1–C 4, –COS–alkyle en C 1–C 4, $-CONH_2$, –CONH–alkyle en C 1–C 4 ou –CONH–CO–alkyle en C 1–C 4, avec un dérivé de la guanidine répondant à la formule VII

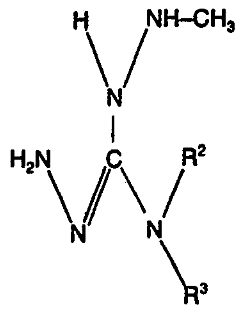

VII

dans laquelle
$R^2$ et $R^3$ ont les significations indiquées dans la revendication 1.

15. Produit pour combattre les croissances de végétaux indésirables et les insectes nuisibles, caractérisé en ce qu'il contient en tant que composant actif, avec des véhicules et/ou d'autres additifs, un composé de formule I selon la revendication 1 ou un composé de formule III selon la revendication 11.

16. Produit selon la revendication 15, caractérisé en ce qu'il contient de 0,1 à 99% en poids d'une substance active de formule I ou III, 99,9 à 1% en poids d'un ou plusieurs additifs et 0 à 25% en poids d'un agent tensioactif.

17. Utilisation d'une substance active selon la revendication 1 dans la lutte contre les insectes nuisibles.

18. Utilisation d'une substance active selon la revendication 1 dans la lutte contre les croissances de végétaux indésirables.

19. Utilisation selon la revendication 18 dans la lutte contre les mauvaises herbes dans les cultures de végétaux utiles, de préférence dans les cultures de céréales, de maïs, de soja et de canne à sucre.

20. Utilisation selon la revendication 19 dans la lutte contre les mauvaises herbes dans les cultures de canne à sucre.

21. Procédé pour combattre les mauvaises herbes dans les cultures de végétaux utiles, caractérisé en ce que l'on applique sur les cultures une quantité efficace d'une substance active selon la revendication 1 ou d'un produit contenant l'un de ces composés.

**Revendications pour l'Etat contractant AT**

1. Procédé de préparation des 4-méthylamino-1,2,4-triazine-5-ones de formule I

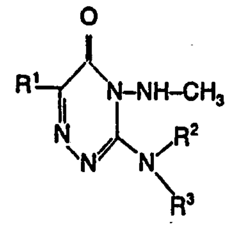

I

dans laquelle
$R^1$ représente un groupe cycloalkyle en C 3–C 8, cycloalkyle en C 3–C 8 substitué par un groupe alkyle en C 1–C 4, halogénocycloalkyle en C 3–C 8, alkyle en C 3–C 8, halogénoalkyle en C 3–C 8, 2-furyle, 2-thiényle, phényle, benzyle ou phényle ou benzyle portant des substituants choisis parmi les halogènes, les groupes alkyle en C 1–C 4, halogénoalkyle en C 1–C 4, alcoxy en C 1–C 4 ou halogéno-alcoxy en C 1–C 4,
$R^2$ représente l'hydrogène ou un groupe méthyle, et
$R^3$ représente un groupe cyclopropyle, cyclopropylméthyle, alkyle en C 1–C 4, alcényle en C 3–C 4 ou alcynyle en C 3–C 4,
étant spécifié que $R^1$, $R^2$ et $R^3$ ne peuvent représenter simultanément: $R^1$ un groupe tert-butyle, $R^2$ l'hydrogène et $R^3$ un groupe méthyle, caractérisé en ce que:

a) on convertit une 4-amino-4H-1,2,4-triazine-5-one de formule II

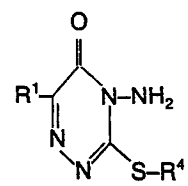

II

dans laquelle $R^1$ a les significations indiquées en référence à la formule I et $R^4$ représente un groupe méthyle ou éthyle, à l'aide d'un agent méthylant, en une 4-méthylamino-4H-1,2,4-triazine-5-one de formule III

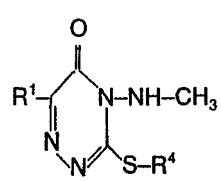

III

qu'on fait réagir à température élevée avec une amine de formule IV

$$H\text{–}NR^2R^3 \qquad \text{IV}$$

dans laquelle $R^2$ et $R^3$ ont les significations indiquées en référence à la formule I, ou bien

b) on fait réagir une 3,4-diamino-4H-1,2,4-triazine-5-one de formule V

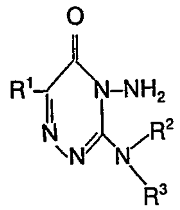

V

dans laquelle $R^1$, $R^2$ et $R^3$ ont les significations indiquées en référence à la formule I, avec un agent méthylant; ou bien

c) on condense un dérivé d'acide α-cétocarboxylique de formule VI

$R^1$–CO–A VI

dans laquelle $R^1$ a les significations indiquées en référence à la formule I, avec un dérivé de la guanidine répondant à la formule VII

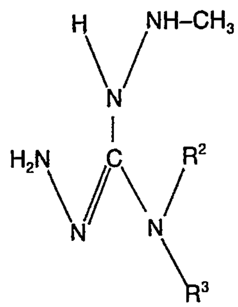

VII

dans laquelle $R^2$ représente l'hydrogène ou un groupe méthyle, et

$R^3$ représente un groupe cyclopropyle, cyclopropylméthyle, alkyle en C 1–C 4, alcényle en C 3–C 4 ou alcynyle en C 3–C 4.

2. Procédé selon la revendication 1, caractérisé en ce que $R^1$ représente un groupe alkyle ramifié en C 3–C 6 ou un groupe halogénoalkyle ramifié en C 3–C 6.

3. Procédé selon la revendication 1, caractérisé en ce que $R^1$ représente un groupe phényle ou un groupe phényle portant des substituants choisis parmi les halogènes, les groupes alkyle en C 1–C 4, halogénoalkyle en C 1–C 4, alcoxy en C 1–C 4 ou halogénoalcoxy en C 1–C 4.

4. Procédé selon la revendication 2, caractérisé en ce que $R^1$ représente un groupe alkyle ramifié en C 3–C 6.

5. Procédé selon la revendication 1, caractérisé en ce que $R^2$ représente l'hydrogène.

6. Procédé selon la revendication 2, caractérisé en ce que $R^3$ représente un groupe alkyle en C 1–C 4.

7. Procédé selon la revendication 6, caractérisé en ce que $R^3$ représente un groupe méthyle.

8. Procédé selon la revendication 1, caractérisé en ce que $R^1$ représente un groupe alkyle ramifié en C 3–C 6 ou halogénoalkyle ramifié en C 3–C 6, $R^2$ représente l'hydrogène ou un groupe méthyle et $R^3$ un groupe alkyle en C 1–C 4.

9. Procédé selon la revendication 1, caractérisé en ce que $R^1$ représente un groupe alkyle ramifié en C 3–C 6, $R^2$ l'hydrogène et $R^3$ un groupe méthyle.

10. Procédé selon la revendication 1, caractérisé en ce que l'on prépare:
la 3,4-bis-(méthylamino)-6-isopropyl-4H-1,2,4-triazine-5-one,
la 3-diméthylamino-4-méthylamino-6-(1,1-diméthyléthyl)-4H-1,2,4-triazine-5-one,
la 3,4-bis-(méthylamino)-6-(1-méthylpropyl)-4H-1,2,4-triazine-5-one,
la 3,4-bis-(méthylamino)-6-phényl-4H-1,2,4-triazine-5-one, ou
la 3,4-bis-(méthylamino)-6-(2-fluorophényl)-4H-1,2,4-triazine-5-one.

11. Produit pour combattre les croissances de végétaux indésirables et les insectes nuisibles, caractérisé en ce qu'il contient en tant que composant actif, avec des véhicules et/ou d'autres additifs, un composé de formule I ou III tel que défini dans la revendication 1.

12. Produit selon la revendication 11, caractérisé en ce qu'il contient de 0,1 à 99% en poids d'une substance active de formule I ou III selon la revendication 1, 99,9 à 1% en poids d'un ou plusieurs additifs et 0 à 25% en poids d'un agent tensio-actif.

13. Utilisation d'une substance active de formule I selon la revendication 1 pour combattre les insectes nuisibles.

14. Utilisation d'une substance active de formule I selon la revendication 1 pour combattre les croissances de végétaux indésirables.

15. Utilisation selon la revendication 14, pour combattre les mauvaises herbes dans les cultures de végétaux utiles, de préférence les cultures de céréales, de maïs, de soja et de canne à sucre.

16. Utilisation selon la revendication 15, pour la lutte contre les mauvaises herbes dans les cultures de canne à sucre.

17. Procédé pour combattre les mauvaises herbes dans les cultures de végétaux utiles, caractérisé en ce que l'on applique sur les cultures une quantité efficace d'une substance active de formule I selon la revendication 1 ou d'un produit contenant un tel composé.